# EUROPEAN PATENT APPLICATION

(11) **EP 4 778 468 A1**
(43) Date of publication of application: **22.07.2026**
(21) Application number: 24865556.5
(22) Date of filing: 13.09.2024
(51) Int. Cl.: A61B 6/50, A61B 5/00, A61B 6/00

(54) **INFORMATION PROCESSING SYSTEM AND INFORMATION PROCESSING METHOD**

(30) Priority: 13.09.2023 JP 2023148672
(71) Applicant: Kyocera Corporation, Kyoto-shi, Kyoto 612-8501 (JP)
(72) Inventor: SHIRATORI, Takaaki, Kyoto-shi, Kyoto 612-8501 (JP); KYOMOTO, Masayuki, Kyoto-shi, Kyoto 612-8501 (JP)
(74) Representative: TBK
(86) International application number: PCT/JP2024/032865
(87) International publication number: WO 2025/058057

(57) **Abstract**

An information processing system includes an information acquisition unit that acquires information on a bone of a subject obtained based on a first method and information on the bone of the subject obtained based on a second method that is different from the first method, and an image generator that generates a display image showing the first information and the second information.

## Description

### TECHNICAL FIELD

The present disclosure relates to an information processing system and an information processing method for supporting understanding of bone states.

### BACKGROUND OF INVENTION

In recent years, estimating or predicting physical information or the like of a subject by using computer calculation has been proposed. For example, Patent Document 1 proposes a method of predicting a future bone density of a postmenopausal woman using a machine learning model.

### CITATION LIST

### PATENT LITERATURE

Patent Document 1: JP 2019-200788 A

### SUMMARY

In an aspect of the present disclosure, an information processing system includes a first acquisition unit that acquires first information indicating information on a bone of a subject obtained based on a first method, and a generator that generates a display image showing the first information together with information on the first method.

In an aspect of the present disclosure, an information processing method includes acquiring first information indicating information on a bone of a subject obtained based on a first method, acquiring second information indicating information on the bone of the subject obtained based on a second method that is different from the first method for the subject, and generating a display image showing the first information and the second information.

In an aspect of the present disclosure, a server apparatus includes a first acquisition unit that acquires first information indicating information on a bone of a subject obtained based on a first method, a second acquisition unit that acquires second information indicating information on the bone of the subject obtained based on a second method that is different from the first method for the subject, and a generator that generates a display image showing the first information and the second information.

In each aspect of the present disclosure, a display apparatus, a first generation apparatus, a second generation apparatus, and a third generation apparatus may be implemented by a computer, and in that case, a control program of the display apparatus, the first generation apparatus, the second generation apparatus, and the third generation apparatus causing a computer to implement as the display apparatus, the first generation apparatus, the second generation apparatus, and the third generation apparatus by operating the computer as each unit (software element) of the display apparatus, the first generation apparatus, the second generation apparatus, and the third generation apparatus, and a computer-readable recording medium on which the control program is recorded are also within the scope of the present disclosure.

### BRIEF DESCRIPTION OF THE DRAWINGS

FIG. 1 is a block diagram illustrating a main part configuration of an example of an information processing system according to a first embodiment.
FIG. 2 is a flowchart showing an example of the flow of a display process of a display apparatus according to the first embodiment.
FIG. 3 is a diagram showing an example of a display image showing inferred values of bone density of a subject at a time point after time points serving as the starting point of inference according to the first embodiment.
FIG. 4 is a diagram showing another example of a display image showing inferred values of bone density of the subject at the time points after the time point serving as the starting point of inference according to the first embodiment.
FIG. 5 is a diagram showing another example of a display image showing inferred values of bone density of the subject at the time points after the time point serving as the starting point of inference according to the first embodiment.
FIG. 6 is a diagram showing an example of a display image showing inferred values of bone density of the subject at time points before a time point serving as the starting point of inference according to the first embodiment.
FIG. 7 is a diagram showing another example of the display image showing inferred values of bone density of the subject at time points before the time point serving as the starting point of inference according to the first embodiment.
FIG. 8 is a diagram showing an example of a display image showing information on the bone density of the subject and transition of the bone density of a reference person according to the first embodiment.
FIG. 9 is a diagram showing an example of a display image showing inferred values in accordance with the change in the timing of starting treatment for the subject according to the first embodiment.
FIG. 10 is a diagram showing an example of a display image showing an error range of inferred values of bone density of the subject according to the first embodiment.
FIG. 11 is a diagram showing an example of a display image showing the inference probability of the inferred value of bone density of the subject according to the first embodiment.
FIG. 12 is a diagram illustrating an example of a display image showing a medical image used in estimation of estimated values and a measurement result corresponding to measured values according to the first embodiment.
FIG. 13 is a diagram illustrating an example of display of a medical image corresponding to an estimated value according to the first embodiment.
FIG. 14 is a diagram illustrating an example of a display image showing a transition of bone density of each of a plurality of sites according to the first embodiment.
FIG. 15 is a set of diagrams illustrating an example of display of a portion where a fracture is likely to occur, included in a display image displayed on a display according to the first embodiment.
FIG. 16 is a block diagram illustrating a main part configuration of an example of an information processing system according to a second embodiment.
FIG. 17 is a block diagram illustrating a main part configuration of an example of an information processing system according to a third embodiment.
FIG. 18 is a block diagram illustrating a main part configuration of an example of an information processing system according to a fourth embodiment.
FIG. 19 is a diagram illustrating an example of a display image showing a subject's fracture risk according to the fourth embodiment.
FIG. 20 is a block diagram illustrating a main part configuration of an example of an information processing system according to a fifth embodiment.
FIG. 21 is a block diagram illustrating a main part configuration of an example of an information processing system according to a sixth embodiment.

### DESCRIPTION OF EMBODIMENTS

### First Embodiment

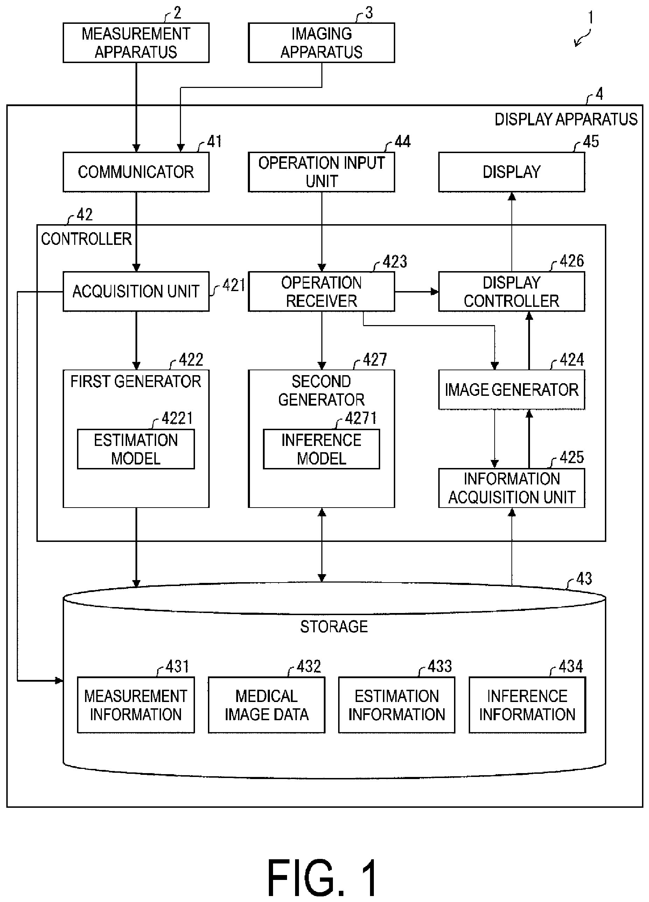

Embodiments of the present disclosure will be described below in detail below with reference to FIGs. 1 to 15.

### Overview

First, an overview of the present embodiment will be described with reference to FIG. 1. FIG. 1 is a block diagram illustrating a main part configuration of an example of an information processing system 1 according to the present embodiment. As illustrated in FIG. 1, the information processing system 1 includes a measurement apparatus 2, an imaging apparatus 3, and a display apparatus (image generation apparatus) 4.

In the present embodiment, the measurement apparatus 2 measures a bone of a subject and transmits first information on the bone of the subject to the display apparatus 4. That is, the display apparatus 4 acquires the first information indicating the information on the bone of the subject obtained based on the measurement of the bone. Here, the first information may be about at least one selected from the group consisting of bone density, bone mass, and bone quality of the subject. Further, as information on a first method, for example, actual measured values, estimated values, inferred values, and the like can be used. Furthermore, the first information may indicate a measured value of bone density of the subject. In the present embodiment, although an example in which the first information is measurement information 431 indicating a measured value of bone density of the subject will be described, the description "bone density" regarding "first information" described in the present disclosure may be appropriately interpreted as at least one selected from the group consisting of "bone density", "bone mass", and "bone quality".

In the present specification, the expression "measurement" may mean "actual measurement". Further, the expression "measured value" may mean "actual measured value".

Although a case in which a subject to which a surgical treatment method is to be applied is a human (that is, a "subject") will be described in an example below, a subject is not limited to a human. That is, the "subject" according to the present disclosure may be, for example, a mammal other than a human, such as an equine, feline, canine, bovine, or porcine mammal. The present disclosure also includes an embodiment in which the "subject," "patient," and "human" are replaced with "animal" as long as any embodiment out of the following embodiments is applicable to any of such animals.

The first information may include information indicating the name of the disease that the subject is suffering from. The information indicating the name of the disease may be information indicating at least one selected from the group consisting of osteoporosis, rheumatism, osteonecrosis (e.g., femoral head osteonecrosis), systemic sclerosis, kidney disease, marble bone disease, and the like. The first information may include bone assessment information. The bone assessment information may include information assessed by using Fracture Risk Assessment Tool (FRAX (trade name)).

The above-described bone density may be a value of the density of the bone. A bone density may be represented by at least one type of bone mineral density per unit area (g/cm²), bone mineral density per unit volume (g/cm³), YAM (%), AGE (%), T-score, and Z-score. YAM is an abbreviation for "Young Adult Mean" and can also be referred to as young adult average percentage, %YAM, YAM ratio, or the like. AGE may be a value relative to an average value of bone density of the same age or the same generation. A T-score is a value obtained by comparing a measured bone density with the young adult average value and dividing the result by the standard deviation of young adults. A Z-score is a value obtained by comparing a measured bone density of the subject with the average value of the same age and dividing the result by the standard deviation of the same age. A bone density is not limited to these examples, and may be, for example, a unique numerical value.

The above-mentioned bone mass is an index related to bone density, and the bone mass is an amount of bone tissues in the skeleton.

For the above-mentioned bone quality, a quality based on, for example, at least one selected from the group consisting of a statistical property of the bone, a geometric property of the bone, a mechanical property of the bone, and a chemical property of the bone can be employed. The bone quality may include information on the subject's attributes to be described below. The bone quality can be based on at least one selected from the group consisting of, for example, a bone metabolic marker, a gender, a race, a menopause status, an age, a state of a cortical bone, a condition of a trabecular bone, a condition of a trabecular bone of cancellous bone, disease information, bone assessment information, medication information, presence or absence of a fracture, the number of fractures, the fractured site, and a history of fractures. More specifically, for example, at least one selected from the group consisting of a bone formation marker, a bone resorption marker, a bone quality marker (for example, a vitamin K value), a cortical bone thickness, a trabecular bone density, a trabecular bone direction, and a trabecular bone score can be employed for the bone quality. The bone quality may be, for example, bone age. As the bone age, for example, a biological maturity of the bone expressed by age can be employed. The bone age may be determined, for example, by the degree of ossification from medical images (e.g., X-ray images or) of hands.

The imaging apparatus 3 transmits medical image data 432, which is data indicating medical images of the subject, to the display apparatus 4.

For the medical images, at least one image selected from the group consisting of an X-ray image, a magnetic resonance imaging (MRI) image, a computed tomography (CT) image, a positron emission tomography (PET) image, and an ultrasound image can be used. The medical images may be images capturing at least a part of the neck, the chest, the waist, the proximal femur, the knee joint, the ankle joint, the shoulder joint, the elbow joint, the wrist joint, the finger joint, or the jaw joint, for example. A site other than the bone may be captured in the X-ray image. For example, in the case of a simple chest X-ray image, the image may include an image of the lungs and an image of the thoracic vertebrae. The X-ray image may be a front image capturing a target site from the front, or may be a side image capturing the target site from a side. A plurality of images may be used for the medical images. For example, for the medical images, a plurality of X-ray images may be used or an X-ray image and a CT image may be used. When a plurality of X-ray images are used, for example, a plurality of front images may be used, a front image and a side image may be used, or a plurality of images capturing different sites may be used.

The display apparatus 4 estimates second information on the bone of the subject using the medical images. Here, the second information may be about at least one selected from the group consisting of bone density, bone mass, and bone quality of the subject. Further, the second information may indicate an estimated value of at least one selected from the group consisting of bone density, bone mass, and bone quality of the subject. That is, the display apparatus 4 acquires, for the subject, the second information indicating the information on the bone of the subject obtained based on a second method different from the first method. Here, the first method may be measurement for the bone of the subject. The second method may be estimation for the bone of the subject.

The second method may be different from the first method in at least one selected from the group including, but not limited to, the measurement site, the calculation method, the calculation conditions, the measurement method, the measurement apparatus, and the measurement conditions. For example, the first method may be measurement performed in a hospital provided with an actual measurement apparatus that performs measurement for bones. The second method may be estimation based on a medical image. The medical image may be captured in a hospital with no actual measurement apparatus provided or may be captured in a hospital provided with an actual measurement apparatus. The first method and the second method may be performed in different hospitals, for example. The first method and the second method can use, for example, measured information, estimated information, and inferred information, but are not limited thereto. The measured information may be, for example, a disease name confirmed by a doctor. The estimated or predicted information may be a disease name estimated or predicted based on various types of information.

In the present embodiment, although an example in which the second information is estimation information 433 indicating an estimated value of bone density of the subject will be described, the description "bone density" regarding "second information" described in the present disclosure may be appropriately interpreted as at least one selected from the group consisting of "bone density", "bone mass", and "bone quality".

Other examples of the first information indicating the information on the bone of the subject obtained based on the measurement of the bone and the second information obtained based on the estimation of the bone are given below.

A case where the bone mainly includes a bone of a spine or an intervertebral disc will be described. In this case, examples of the first information and the second information include information indicating at least one selected from the group consisting of vertebral height, intervertebral distance, anterior disc space distance, posterior disc space distance, poor posture classification, ossification occupancy rate (ratio of the thickness of ossified ligament to anterior-posterior diameter of the spinal canal), residual effective anterior-posterior diameter (thickness between anterior-posterior diameter of the spinal canal and ossified ligament), posterior vertebral slip distance (distance between the posterior inferior angle of the superior vertebral body and the tip of the inferior vertebral arch), and Meyerding classification.

Examples of diseases that can be diagnosed based on the above information include degenerative spondylosis, vertebral body fractures (including traumatic and osteoporotic fractures), spinal stenosis, hernias, degenerative spondylolisthesis, scoliosis, degenerative diseases of the thoracic and lumbar spine, bilateral congenital hip dislocation, ossification of spinal ligaments, etc.

A case in which the bone mainly includes a hip bone will be described. In this case, examples of the first information and the second information include information indicating at least one selected from the group consisting of a joint space area, a joint space distance (width), a minimum joint space distance (width), a CE angle (Center Edge angle: an angle formed by a perpendicular line passing through the femoral head center and a line connecting the femoral head center and the lateral acetabular rim), a Sharp angle (Acetabular Angle: an angle formed by a line connecting the lateral acetabular margin and the tip of the lacrimal fossa and the line connecting both lacrimal fossae), ARO (Acetabular Roof Obliquity: angle formed by the horizontal line passing through the acetabular floor and the line connecting the lateral acetabular margin and the acetabular floor), AHI (Acetabular Head Index: distance from the medial end of the femoral head to the lateral end of the acetabulum divided by the transverse diameter of the femoral head), and Kellgren-Lawrence (KL) classification. At least one selected from the group consisting of an actual measured value, an estimated value, and an inferred value can be used as the first information and the second information.

Examples of diseases that can be diagnosed based on the above information include osteoarthritis of the hip (OA), femoroacetabular impingement (a condition associated with OA), hip dysplasia, hip dislocation (including congenital), femoral slippage, femoral head necrosis, etc.

A case in which the bone mainly includes a knee bone will be described. In this case, examples of the first information and the second information include information indicating at least one selected from the group consisting of the joint space area, joint space distance (width), minimum joint space distance (width), osteophyte area, femorotibial angle (lateral femorotibial angle), Osteoarthritis Research Society International (OARSI) classification, or Kellgren-Lawrence (KL) classification. The information enables, for example, diagnosis of osteoarthritis of the knees (OA).

A case in which the bone mainly includes foot bones will be described. In this case, the first information and the second information may be information indicating at least one of a talar tilt angle and anterior talar drawer distance. Examples of diseases that can be diagnosed based on the above information include ankle sprain, talar osteonecrosis, rheumatoid arthritis, and ankle impingement syndrome.

The display apparatus 4 generates a display image that shows the first information and the second information. In the present embodiment, an example in which the display apparatus 4 generates a display image showing a measured value of the bone density of the subject indicated by the measurement information 431 and an estimated value of the bone density of the subject indicated by the estimation information 433 will be described.

According to the above configuration, the information processing system 1 can generate a display image that displays information on the bone of the subject obtained based on a plurality of different methods, for example, the measurement and estimation methods. That is, in the medical field, a transition of a bone state of a subject can be ascertained by using estimation or the like which is a method different from measurement without periodically performing measurement using a predetermined measurement apparatus for the bone of the subject. For example, the transition of the bone state of the subject may be a transition of at least one selected from the group consisting of bone density, bone mass, and bone quality of the subject.

The first information and the second information may be information of the same item indicating the state of the bone or may be information of different items. For example, the first information and the second information may be information of items having a correspondence relationship. Specifically, the first information and the second information may be information of items associated with each other due to unit conversion, a conversion formula, or the like.

A measured value of the subject displayed in the image may be a measured value of at least one selected from the group consisting of the bone density, bone mass, and bone quality of the subject measured at a first time point. An estimated value of the subject displayed in the image may be an estimated value estimated by an estimation model 4221 or the like included in the display apparatus 4 from a medical image in which the bone of the subject at a second time point, which is different from the first time point, is shown. The estimated value may be an estimated value of at least one selected from the group consisting of bone density, bone mass, and bone quality. The display apparatus 4 may generate a display image in which measured values and estimated values of the subject are arranged in chronological order of the first time point and the second time point.

According to the above-described configuration, the information processing system 1 generates an image in which the information on the bone of the subject at the measured first time point and the information on the bone of the subject at the estimated second time point are arranged in chronological order based on the first time point and the second time point. That is, the information processing system 1 can generate an image in which measured values and estimated values of at least one selected from the group consisting of the bone density, the bone mass, and the bone quality of the subject are arranged in chronological order. Accordingly, the information processing system 1 can improve the convenience of the user when physical information or the like of the subject is estimated or inferred.

### Information Processing System 1

The information processing system 1 according to the present embodiment will be described in detail. As described above, the information processing system 1 includes the measurement apparatus 2, the imaging apparatus 3, and the display apparatus 4.

### Measurement Apparatus 2

The measurement apparatus 2 measures a bone density of a subject. The measurement apparatus 2 may be any apparatus that can measure a bone density of a subject, and is not particularly limited, but the following can be given as an example.

For example, the measurement apparatus 2 may measure a bone density by using a dual-energy X-ray absorptiometry (DXA) method, ultrasonography, or a micro densitometry (MD) method.

The measurement apparatus 2 transmits measurement information 431 indicating a measured value of the bone density of the subject to the display apparatus 4. That is, the measurement information 431 can be rephrased as information measured using a predetermined measurement apparatus that performs measurement on a bone. The measurement information 431 may include information indicating the date and time when the measurement was performed. Communication between the measurement apparatus 2 and the display apparatus 4 may be wired communication or wireless communication. Further, the communication may be communication through the Internet.

### Imaging Apparatus 3

The imaging apparatus 3 may be, for example, an apparatus that performs imaging to acquire information on the inside of the body of a subject. The imaging apparatus 3 captures medical images of a subject. A medical image captured by the imaging apparatus 3 may be an image including information of a bone of a subject, and is not particularly limited, but the following examples can be given. For example, the medical image may be an X-ray image, an MRI image, a CT image, a PET image, or an ultrasound image.

The imaging apparatus 3 transmits medical image data 432, which is data indicating captured medical images, to the display apparatus 4. The medical image data 432 may include information indicating the date and time when the medical image was captured. Communication between the imaging apparatus 3 and the display apparatus 4 may be wired communication or wireless communication. Further, the communication may be communication through the Internet. Note that, when the imaging apparatus 3 is not able to directly communicate with the display apparatus 4, the medical image data 432 may be moved using a portable memory or the like.

### Display Apparatus 4

The display apparatus 4 generates a display image showing a measured value of the bone density of the subject and an estimated value of the bone density of the subject, and displays the image. The display apparatus 4 includes a communicator 41, a controller 42, a storage 43, an operation input unit 44, and a display 45.

### Communicator 41

The communicator 41 communicates with the measurement apparatus 2 and the imaging apparatus 3. The communicator 41 receives the measurement information 431 from the measurement apparatus 2. The communicator 41 receives the medical image data 432 from the imaging apparatus 3.

### Controller 42

The controller 42 comprehensively controls each unit of the display apparatus 4. The controller 42 includes an acquisition unit 421 (first acquisition unit), a first generator 422, an operation receiver 423, an image generator (generator) 424, an information acquisition unit (first acquisition unit, second acquisition unit, third acquisition unit) 425, a display controller 426, and a second generator 427.

### Acquisition Unit 421

The acquisition unit 421 acquires the measurement information 431 from the measurement apparatus 2 via the communicator 41. The acquisition unit 421 acquires the medical image data 432 from the imaging apparatus 3 via the communicator 41.

The acquisition unit 421 stores the acquired measurement information 431 and medical image data 432 in the storage 43. The acquisition unit 421 transmits the acquired medical image data 432 to the first generator 422.

### First Generator 422

The first generator 422 generates estimation information 433 indicating an estimated value of the bone density of the subject estimated from a medical image (first image) indicated by the medical image data 432 of the subject received from the acquisition unit 421. The first generator 422 stores the generated estimation information 433 in the storage 43. The first generator 422 includes the estimation model 4221.

### Estimation Model 4221

The estimation model 4221 is for estimating the bone density of the subject from a medical image indicated by the medical image data 432 of the subject. The estimation model 4221 may be set based on learning data including a medical image (second image) showing a bone of a first person and training data including a value of at least one selected from the group consisting of bone density, bone mass, and bone quality of the first person. That is, the estimation model 4221 is a trained model.

For example, the bone density of a person having a bone captured in a simple X-ray image for learning indicated by corresponding each of a plurality of pieces of learning image data included in learning data may be associated, as training data, with the learning image data. The bone density of the training data may be a bone density obtained by using the DXA method, the ultrasound method, or the MD method. The "first person" mentioned above may not be particularly limited to humans. For example, it may be an animal of the same species as the "subject".

### Operation Receiver 423

The operation receiver 423 receives an operation input by the user via the operation input unit 44. The operation receiver 423 receives an operation input to instruct display of a display image showing information on a bone of a subject. Upon receiving a corresponding operation input, the operation receiver 423 instructs the image generator 424 to generate an image.

The operation receiver 423 receives an operation input to instruct estimation for a bone of a subject. Upon receiving a corresponding operation input, the operation receiver 423 instructs the second generator 427 to generate the inference information 434 indicating estimation for the bone of the subject. Further, the operation receiver 423 instructs the image generator 424 to generate a display image showing the inference information 434.

The operation receiver 423 receives an operation input to instruct display termination. Upon receiving the operation input, the operation receiver 423 instructs the display controller 426 to terminate the display.

### Image Generator 424

When receiving an instruction to generate a display image showing information on a bone of a subject, the image generator 424 acquires the measurement information 431 and the estimation information 433 in the storage 43 through the information acquisition unit 425. The image generator 424 generates a display image showing information on the bone of the subject using the measurement information 431 and the estimation information 433.

Upon receiving an instruction to generate a display image showing the inference information 434, the image generator 424 acquires the measurement information 431, the estimation information 433, and the inference information 434 in the storage 43 through the information acquisition unit 425. The image generator 424 generates a display image showing information on the bone of the subject using the measurement information 431, the estimation information 433 and the inference information 434.

The image generator 424 transmits data of the generated display image to the display controller 426. An example of the display image generated by the image generator 424 will be described in detail below.

### Information Acquisition Unit 425

The information acquisition unit 425 acquires the measurement information 431 and the estimation information 433 stored in the storage 43 in response to an instruction from the image generator 424, and transmits the acquired information to the image generator 424.

The information acquisition unit 425 acquires the measurement information 431, the estimation information 433, and the inference information 434 stored in the storage 43 in response to an instruction from the image generator 424, and transmits the acquired information to the image generator 424.

### Display Controller 426

The display controller 426 causes the display 45 to display a display image indicated by the image data received from the image generator 424.

The display controller 426 terminates image display of the display 45 in response to an instruction to terminate the display received from the operation receiver 423.

### Second Generator 427

The second generator 427 generates the inference information 434 indicating an inferred value of a bone density of a subject inferred based on a medical image indicated by the medical image data 432 stored in the storage 43.

For example, the second generator 427 may generate the inference information 434 on at least one selected from the group consisting of the bone density, bone mass, and bone quality inferred based on a medical image capturing the bone of the subject at a first time point at which at least one selected from the group consisting of the bone density, bone mass, and bone quality of the subject was estimated. In the present embodiment, although an example in which the inference information 434 indicates an inferred value of a bone density of a subject is described, the phrase "inference information 434" described in the present disclosure may be appropriately interpreted as information indicating at least one selected from the group consisting of a "bone density", "bone mass", and "bone quality".

Here, the measurement of the bone density at the first time point and the capturing of the medical image capturing the bone of the subject at the first time point may not be performed exactly at the same time. For example, the measurement and the capturing may be performed within a predetermined time period. The predetermined time period is not particularly limited as long as it is a time period in which a significant change in the bone density of the subject is unlikely to occur, and examples thereof include half a day, one day, one week, one month, three months, and six months. When no medical image capturing the bone of the subject is captured at the first time point, the display apparatus 4 may not receive the medical image data 432 indicating a medical image capturing the bone of the subject at the first time point. That is, when a medical image capturing the bone of the subject is captured at the first time point, the display apparatus 4 may receive the medical image data 432 indicating a medical image capturing the bone of the subject at the first time point. In the present specification, the time point at which the medical image used for generating the inference information 434 is captured is referred to as a starting point for inference. The inference information 434 may indicate an inferred value of the bone density of the subject at a third time point. The third time point may be a time point different from the first time point.

The second generator 427 may generate the inference information 434 on the bone density of the subject inferred from a medical image capturing the bone of the subject at the second time point used by the first generator 422 to estimate an estimated value of the bone density of the subject. The inference information 434 may indicate an inferred value of the bone density of the subject at the third time point. The third time point may be a time point different from the second time point.

When the image generator 424 generates an image using the inference information 434, the image generator 424 may generate a display image showing the inference information 434 associated with the third time point.

The second generator 427 may generate the inference information 434 in response to an instruction to generate the inference information 434 indicating inference for the bone of the subject received from the operation receiver 423. The second generator 427 stores the generated inference information 434 in the storage 43. The second generator 427 includes an inference model 4271.

### Inference Model 4271

The inference model 4271 infers, based on a medical image indicated by the medical image data 432 of the subject, the bone density of the subject at a time point that is different from the time point at which the medical image was captured. The inference model 4271 may be set based on learning data including a medical image (third image) showing a bone of a second person and training data including a bone density of the second person. The "second person" mentioned above may not be particularly limited to humans. For example, it may be an animal of the same species as the "subject".

For example, the bone density of a person having a bone captured in a simple X-ray image for learning indicated by corresponding a plurality of pieces of learning image data included in learning data is associated, as training data, with the learning image data. The training data may be associated with each of the plurality of pieces of learning image data, or a plurality of pieces of learning image data associated with one piece of training data may be prepared. The training data may be a bone density obtained by using the DXA method, the ultrasound method, or the MD method. Alternatively, a value estimated or inferred from another trained model may be used in the training data.

As the training data, data measured at any time can be used. The training data may be measured at the same time as the time when the image data for learning is captured, or may be measured on a different time axis. For example, in the measurement of the training data, a predetermined criterion for the time when the image data for learning is captured may be set. As the predetermined criterion, for example, a period within three months, six months, or one year from the past can be adopted for the time when the image data for learning is captured. A predetermined criterion of the training data may be at least one selected from the group consisting of the presence or absence of treatment, age, menopause status, and gender of the subject in the training data. The treatment may involve at least one selected from the group consisting of a drug, nutritional guidance (including the regular use of supplements), and exercise guidance.

The image data for learning of the learning data may be a series of image data obtained by capturing the same person on different time axes. That is, the image data for learning may include the following first learning data and second learning data. The first learning data may include an X-ray image of a bone of a certain person. The second learning data may include an image of the same person as the certain person, which is an X-ray image being captured after that of the first learning data.

The image data for learning of the learning data may be a data group in which the same sites of different persons of different ages have been taken. The image data for learning of the learning data may be a series of image data obtained by capturing the same person on different time axes. The image data for learning of the learning data may be a series of image data obtained by capturing the same sites on different time axes.

The inference information 434 generated by the second generator 427 may be related to the bone density of the subject when treatment with respect to the bone density was performed on the subject. In this case, the second person described above is a person who has received treatment for the bone density, and the inference model 4271 may be set based on learning data including a medical image capturing a bone of the second person who has received the treatment and training data including a value of the bone density of the second person. For example, the learning data described above may include medical images before and after treatment. The above-described training data may include measured values of a bone density before and after treatment.

The second generator 427 may include the following two models as the inference model 4271. (1) A model set based on learning data including a medical image capturing a bone of the second person who has received treatment and training data including a measured value of the bone density of the second person who has received treatment. (2) A model set based on learning data including a medical image capturing a bone of the second person who has not received treatment and training data including a measured value of the bone density of the second person who has not received treatment. The treatment may include treatment with medication (for example, also referred to as drug treatment), nutritional guidance, exercise guidance, and the like.

The second generator 427 may generate the inference information 434 according to the timing at which the treatment of the subject is started.

When the medical image indicated by the medical image data 432 to be used for inference of the bone density of the subject is an image at the first time point, the third time point may be a time point before the first time point or a time point after the first time point. When the medical image indicated by the medical image data 432 to be used for inference of the bone density of the subject is an image at the second time point, the third time point may be a time point before the second time point or a time point after the second time point.

The medical image (second image) showing the bone of the first person, which is the learning data of the estimation model 4221, and the medical image (third image) showing the bone of the second person, which is the learning data of the inference model 4271, may be the same image.

The second image and the third image may be medical images obtained by capturing different sites. The person captured in the second image and the person captured in the third image may be the same person. The person captured in the second image and the person captured in the third image may be different persons.

The training data including the measured value of the bone density of the second person may be data measured at a time point different from the time point at which the third image was captured. For example, the training data may be at least one selected from the group consisting of data six months, one year, and several years after the third image was captured, and data several years before the third image was captured. The learning data may be a measured value of bone density measured in a predetermined time period including a time point at which the third image is captured.

### Storage 43

The storage 43 stores the measurement information 431, the medical image data 432, the estimation information 433, and the inference information 434 described above. The storage 43 may store subject data indicating information on the subject. The subject data may include data indicating whether or not the subject has been treated, a period of the treatment performed on the subject, details of the treatment performed on the subject, the date and time when the subject is injured, and the like.

### Operation Input Unit 44

The operation input unit 44 receives operation input from a user. For example, the operation input unit 44 may be integrated with the display 45. The display 45 may display instructions for the process of inferring the bone density of the subject, an image generation process, the image display process, and the like, which are processes performed by the controller 42. The user may select these display areas to perform processing corresponding to the display areas.

### Display 45

The display 45 displays the image generated by the image generator 424 to the user. Examples of the display 45 include a display, a monitor, and the like.

### Flow of Display Process

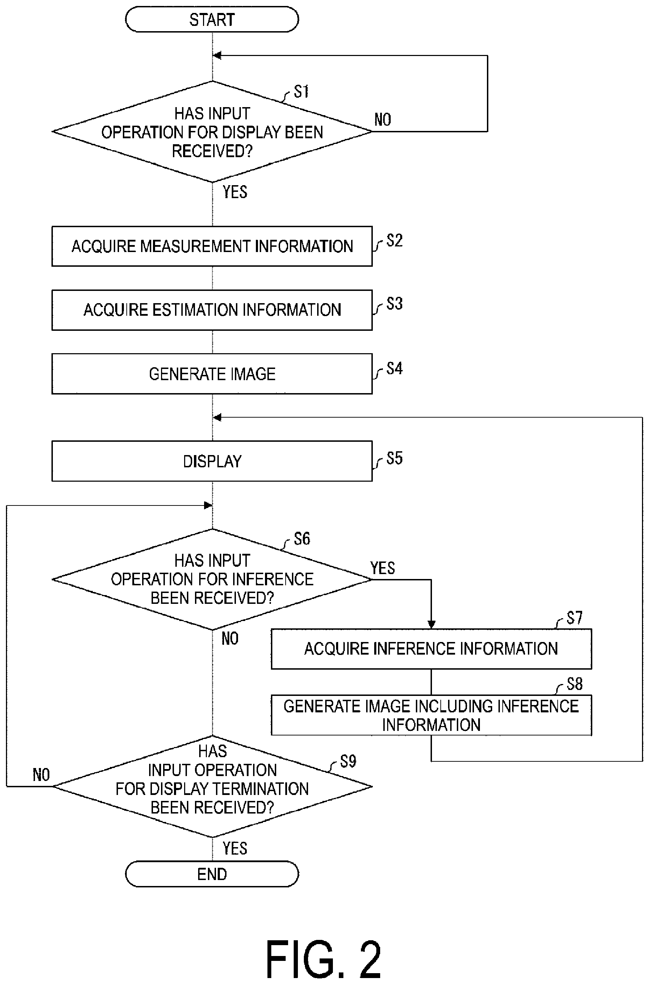

The flow of a display process of the display apparatus 4 will be described with reference to FIG. 2. FIG. 2 is a flowchart showing an example of the flow of the display process of the display apparatus 4. As shown in FIG. 2, when the operation receiver 423 receives an operation input to instruct display of a display image showing information on a bone of a subject (YES in S1), the information acquisition unit 425 performs the following process.

The information acquisition unit 425 acquires the measurement information 431 (S2: first acquisition step). The information acquisition unit 425 acquires the estimation information 433 (S3: second acquisition step). For example, the first information may indicate the bone density of the subject measured at the first time point, and the second information may indicate the bone density estimated by using an estimation model from a first image capturing the bone of the subject at the second time point different from the first time point. Subsequently, the image generator 424 generates a display image showing the measured value indicated by the measurement information 431 and the estimated value indicated by the estimation information 433 (S4: generation step). Subsequently, the display controller 426 causes the display 45 to display the display image generated by the image generator 424 (S5).

When the operation receiver 423 receives an operation input to instruct inference about the bone of the subject (YES in S6), the information acquisition unit 425 acquires the inference information 434 (S7). The operation input may include an input indicating the time point serving as the starting point of the inference and an input indicating when the inference was performed from the time point serving as the starting point. For example, although not particularly limited, examples of the inference include inference a half year after the time point serving as the starting point, inference one year after the time point serving as the starting point, inference a half year before the time point serving as the starting point, inference one year before the time point serving as the starting point, and the like. The operation input may include an input regarding whether the subject received treatment or not. Examples of the inference include inference when the subject has been treated, inference when the subject has not been treated, inference when the subject has been treated and when the subject has not been treated, and the like. The operation input may include an input regarding the timing when treatment for the subject starts. For example, the inference may be in accordance with the timing when treatment for the subject starts. Alternatively, when treatment was performed, the inference may be performed a plurality of times depending on the details of the treatment. For example, for the inference, at least one selected from the group consisting of inference of drug treatment, inference of nutritional guidance treatment, and inference of exercise guidance treatment may be performed.

Subsequently, the image generator 424 generates a display image showing the inferred value indicated by the inference information 434 (S8). Then, the process returns to S5.

When the operation receiver 423 has not received the operation input to instruct inference for the bone of the subject (NO in S6) but received an operation input to instruct display termination (YES in S9), the process is terminated.

When the operation receiver 423 has not received the operation input for instructing the display of the display image showing the information on the bones of the subject (NO in S1), the process in S1 is repeated.

When the operation receiver 423 has neither received the operation input to instruct inference for the bone of the subject (NO in S6) nor received an operation input to instruct display termination (NO in S9), the process returns to S6.

### Example of Display Image Generated by Image Generator 424

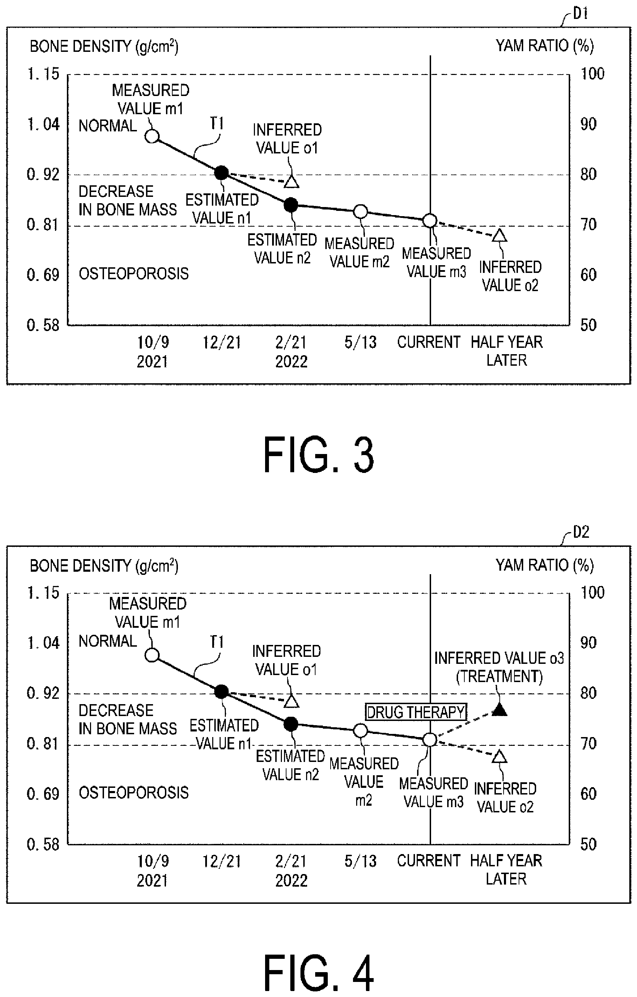

Examples of display images generated by the image generator 424 will be described with reference to FIGs. 3 to 9. The description "bone density" shown in FIGs. 3 to 9 may be appropriately interpreted as at least one selected from the group consisting of "bone density", "bone mass", and "bone quality".

Example of Display Image Showing Inferred Value at Time Point After Time Point Serving as Starting Point of Inference

First, an example of a display image showing an inferred value at a time point after the time point serving as the starting point of inference will be described. FIGs. 3 to 5 are diagrams showing examples of display images displaying inferred values of the bone density of a subject at a time point after the time point serving as the starting point of inference.

The image D1 shown in FIG. 3 shows measured values m1 to m3 of the bone density of the subject at the time points of the measurement, estimated values n1 and n2 of the bone density of the subject at the time points at which the medical image was captured, and a transition T1 of the bone density based on the measured values m1 to m3 and the estimated values n1 and n2. The name of the facility where the medical image was captured may be displayed in the image D1.

The display mode for the measured values of the bone density of the subject may be different from the display mode for the estimated values of the bone density of the subject. In the different display modes, for example, the measured values may be indicated by white circles. The estimated values may be indicated by black circles.

The inferred values o1 and o2 are displayed in the image D1. The measured values, the estimated values, and the inferred values may be displayed in different display modes. In the example shown in FIG. 3, the inferred values o1 and o2 are indicated by white triangles. The inferred values are shown to be connected to dotted lines, and the inferred values are indicated at the end points of the dotted lines. The starting points of the dotted lines indicate the time points serving as the starting point of inference.

In the image D1, the inferred value o1 has the time point of Dec. 21, 2021 as the starting point of the inference. The inferred value o1 was inferred based on the medical image captured at the time point of Dec. 21, 2021. The inferred value o1 is of the bone density of the subject inferred at the time point of Feb. 21, 2022, which is two months after the starting point.

The inferred value o2 has the current time set as the starting point of the inference, and was inferred based on the medical image captured at the current time. The inferred value o2 is of the bone density of the subject inferred at a time point half a year after the current time.

When the bone density of the subject is measured at a certain time point, the measured value at the certain time point displayed in the image D1 is indicated by the corresponding measurement information 431 obtained from the measurement. On the other hand, when the same time point as the certain time point is set as the starting point of inference, the inferred value with the certain time point displayed in the image D1 as the starting point of the inference is inferred from the medical image captured at the certain time point.

Each measured value, estimated value, and inferred value are displayed with an indication of what type of the value is. Specifically, each measured value, estimated value, and inferred value may be displayed with indications of "measured value", "estimated value", and "inferred value". The indications "measured value", "estimated value", and "inferred value" may be displayed in different display modes, respectively. By making the display modes different from each other in this manner, the user can easily recognize that the values are based on different methods. The different display modes may be represented by, for example, differences in the size, font, and color of characters. The "measured value", "estimated value", and "inferred value" may be displayed in display modes in which at least icons, abbreviations, markers, and the like are different. Information on the method for obtaining values to be displayed may be notated in the image D1. Examples of the method include "measurement", "estimation", and "inference". That is, the image generator 424 may generate a display image including display indicating the first method and the second method. The indications of methods as described above can improve user convenience. A Young Adult Mean (YAM) ratio, which is a value corresponding to a bone density, is displayed in the image D1. The YAM ratio is a value indicating a diagnostic criterion, and is displayed in the image D1 with indications "normal" if the YAM ratio is in a range of 100% to 80%, "decrease in bone mass" if the YAM ratio is in a range of 80% to 70%, and "osteoporosis" if the YAM ratio is in a range lower than 70%.

The image D1 may display at least one selected from the group consisting of a bone mineral density per unit area (g/cm²), a bone mineral density per unit volume (g/cm³), AGE, T-score, and Z-score. For AGE, for example, a value relative to an average value of bone density of the same age or the same generation may be displayed. The image D1 may be configured such that those values make it easier to recognize a category corresponding to a predetermined criterion. For the categories, "normal", "decrease in bone mass", "osteoporosis", and the like according to these values can be used. When a T-score is displayed in the image D1, for example, a T-score of -1.0 or greater may indicate 'normal bone density', a T-score of -1.0 to -2.5 may indicate 'low bone density' or 'osteopenia', and a T score of -2.5 or less may indicate 'osteoporosis'. When a Z-score is displayed in the image D1, a Z-score lower than -2.0 may indicate low in bone density compared to the peer age group, and a Z-score exceeding -2.0 may indicate high in bone density compared to the peer age group.

The second generator 427 may infer the time at which the bone density of the subject reaches a state of "normal", "low bone density or osteopenia", or "osteoporosis" in terms of YAM ratio, T-score, or Z-score. That is, the second generator 427 may generate the inference information 434 indicating the time. As an example, the inference information 434 indicates that the bone density of the subject will reach 70% or less of the YAM ratio (osteoporosis) in one year, or the like.

That is, the image generator 424 generates a display image that displays information indicating the determination criterion for bone density. The information indicating the determination criterion may indicate a determination criterion according to the age of the subject. The information indicating the determination criterion may indicate a determination criterion based on a unique criterion, an already-known guideline, or the like.

Example 1 Showing Inferred Value for Subject Who Received Treatment An example of a display image showing an inferred value of bone density of a subject when the subject received treatment will be described with reference to FIG. 4. FIG. 4 is a diagram showing an example of a display image showing inferred values of the bone density of a subject when the subject received treatment. The image D2 shown in FIG. 4 shows measured values m1 to m3 of the bone density at the time points of the measurement, estimated values n1 and n2 of the bone density of the subject at the time points at which the medical image was captured, and a transition T1 of the bone density based on the measured values and the estimated values. The same representations in the image D2 as those in the above-described image D1 are denoted by the same reference signs, and descriptions thereof will not be repeated.

The image D2 shows the inferred value o1, inferred value o2, and inferred value o3. The inferred value o1 and inferred value o2 shown in the image D2 are inferred values when the subject has not received treatment.

The inferred value o3 is an inferred value when the subject received treatment. In the image D2, the indication "drug therapy" is indicated at the current time point. The indication indicates that "drug therapy" started from the current time point. That is, the inferred value o3 is an inferred value obtained in half a year when "drug therapy", which is treatment for the subject, was started from the current time point.

In the example shown in FIG. 4, the inferred values o1 and o2, which are inferred values when the subject has not received treatment, are indicated by white triangles. The inferred value o3, which is an inferred value when the subject has received treatment, is indicated by a black triangle.

According to the above-described configuration, the user can be caused to visually recognize the effectiveness of the treatment.

In the present example, an example of a display image showing inferred values in half a year when "drug therapy", treatment for the subject is started from the current time point has been described. On the other hand, the image may show inferred values of the bone density of the subject when the subject received treatment at a time point before the current time point, that is, at a past time point. Inferred values for a plurality of drugs used in the drug therapy may be shown in the display image. More specifically, for example, inferred values of each of a plurality of drugs (for example, three types) used for the drug therapy may be shown in the display image. In this case, with respect to a plurality of drugs, inferred values of each of a few top-ranking drugs (for example, three types) having highest therapeutic effects among many drugs (for example, ten types) may be shown in the display image.

### Example 2 Showing Inferred Value for Subject Who Received Treatment: with Display of Treatment Period

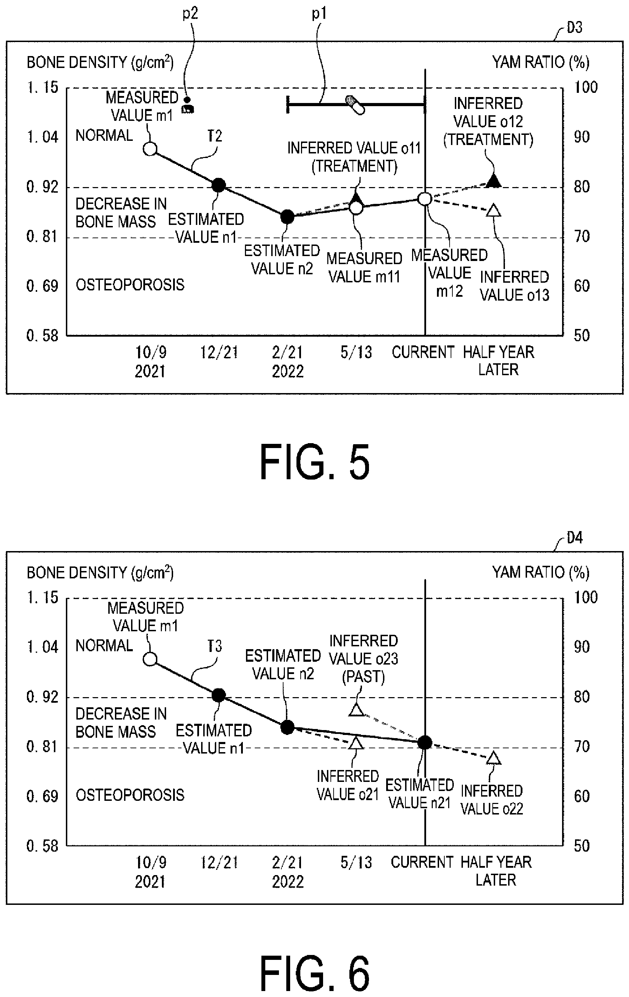

Another example of the display image showing an inferred value of the bone density of a subject when the subject received treatment will be described with reference to FIG. 5. FIG. 5 is a diagram showing another example of the display image showing inferred values of the bone density of the subject when the subject received treatment. The image D3 shown in FIG. 5 shows measured value m1 and measured values m11 and m12 of the bone density at the time points of the measurement, estimated values n1 and n2 of the bone density of the subject at the time points at which the medical image was captured, and a transition T2 of the bone density based on the measured values and the estimated values. The same representations in the image D3 as those in the above-described image D1 and image D2 are denoted by the same reference signs, and descriptions thereof will not be repeated.

In the image D3, a representation p1 indicating a period of treatment actually performed for the subject is shown. The representation p1 represents the period of treatment from Feb. 21, 2022 to the current time point. In other words, the image generator 424 may generate a display image showing information indicating a period of treatment for the bone density of the subject. Information indicating details of the treatment may be shown in the image D3. A representation p2 indicating the date and time when the subject was injured the subject was injured is shown in the image D3. The image generator 424 may generate a display image showing the representation p1, representation p2, and the like, which are information on the subject, using subject data stored in the storage 43. The display apparatus 4 may receive an operation input for selecting whether the display of the representation p1 indicating the period of treatment and the representation p2 indicating the date and time when the subject was injured is necessary or not.

The image D3 shows the inferred value o11, inferred value o12, and inferred value o13. The inferred value o11 is an inferred value when the subject received treatment at the time point when the treatment of Feb. 21, 2021 was started as the starting point of the inference. The inferred value o11 is an inferred value at the time point of May 13, 2021, which is a time point after the starting point.

The inferred value o12 is an inferred value after half a year from the current time point set as the starting point of the inference if the subject is not subjected to the treatment from then on.

The inferred value o13 is an inferred value after half a year from the current time point as the starting point of the inference if the subject continuously receives the treatment from then on.

### Example of Display Image Showing Inferred Value at Time Point Before Time Point Serving as Starting Point of Inference

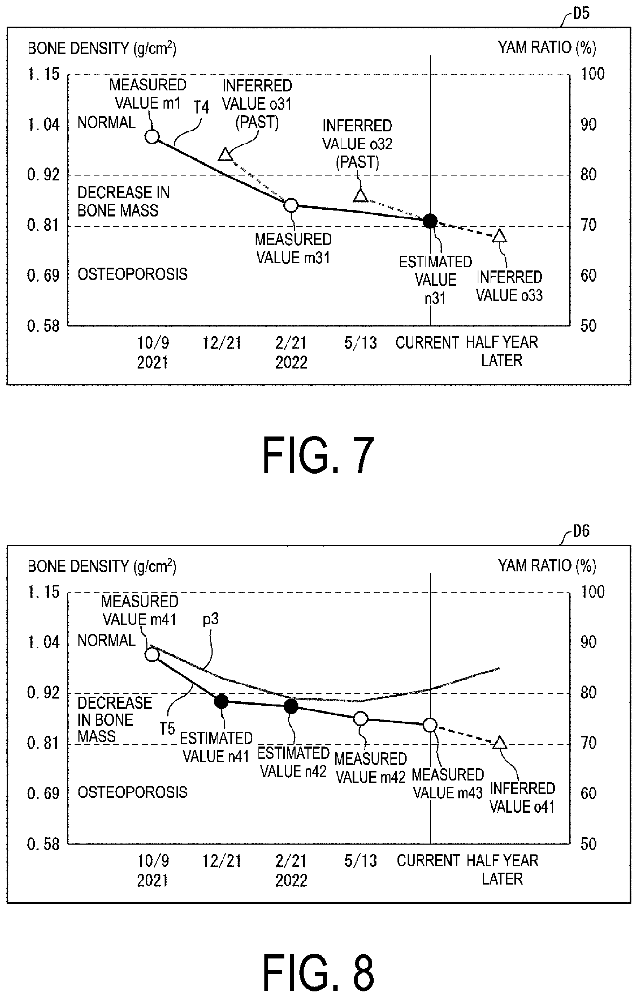

An example of a display image showing an inferred value at a time point before the time point serving as the starting point of inference will be described. FIGs. 6 and 7 are diagrams showing examples of display images showing inferred values of the bone density of a subject at a time point before the time point serving as the starting point of inference.

Example of Display Image Showing Inferred Values of Bone Density prior to Current Time Point When Starting Point of Inference is Current Time Point The image D4 shown in FIG. 6 shows the measured value m1 of the bone density of the subject at the time points of the measurement, estimated values n1, n2, and n21 of the bone density of the subject at the time points at which the medical image was captured, and a transition T3 of the bone density based on the measured value and the estimated values. The image D4 shows the inferred value o21, inferred value o22, and inferred value o23. The same representations in the image D4 shown in FIG. 6 as those in the above-described image D1 to image D3 are denoted by the same reference signs, and descriptions thereof will not be repeated.

The inferred value o21 is an inferred value at the time point of May 13, 2022, which is a time point after the starting point of the inference, which is the time point of Feb. 21, 2022.

The inferred value o22 is an inferred value at a time point in half a year from the starting point of the inference when the current time point is set as the starting point.

The inferred value o23 is an inferred value at the time point of May 13, 2021 that is before the current time point set as the starting point of the inference. That is, the inferred value o23 is an inferred value prior to the current time point serving as the starting point of the inference.

### Example of Display Image Showing Inferred Values of Bone Density prior to Starting Point of Inference When Starting Point is Time Point of Past

The image D5 shown in FIG. 7 shows the measured value m1 and the measured value m31 of the bone density of the subject at the time points of the measurement, the estimated value n31 of the bone density of the subject at the time point at which the medical image was captured, and a transition T4 of the bone density based on the measured values and the estimated value. The image D5 shows the inferred value o31, inferred value o32, and inferred value o33. The same representations in the image D5 shown in FIG. 7 as those in the above-described image D1 to image D4 are denoted by the same reference signs, and descriptions thereof will not be repeated.

The inferred value o31 is an inferred value at the time point of Dec. 21, 2021, which is a time point before the starting point of the inference, which is the time point of Feb. 21, 2022. That is, the inferred value o31 is an inferred value at a time point that is even further in the past than the starting point of the inference.

The inferred value o32 is an inferred value at the time point of May 13, 2022 that is before the current time point set as the starting point of the inference.

The inferred value o33 is an inferred value at a time point in half a year from the current time point set as the starting point of the inference.

According to the examples illustrated in FIGs. 6 and 7, the value of the bone density of the subject at a past time point at which no medical image was captured or no bone density was measured can be compensated for. In a medical institution, the value of the bone density of the subject at a past time point can be inferred at the time of the first medical examination of the subject.

### Example of Display Image Showing Transition Information Indicating Transition of Bone Density of Reference Person Different from Subject

An example of a display image showing information on the bone density of a subject and transition information indicating a transition of the bone density of a reference person different from the subject will be described. FIG. 8 is a diagram showing an example of a display image showing information on the bone density of a subject and transition information indicating a transition of the bone density of a reference person.

The image D6 shown in FIG. 8 shows the measured values m41 to m43 of the bone density of the subject at the time points of the measurement, estimated values n41 and n42 of the bone density of the subject at the time points at which the medical image was captured, and a transition T5 of the bone density of the subject based on the measured values and the estimated values. The image D6 shows an inferred value o41. The inferred value o41 is an inferred value of the bone density of the subject at a time point in half a year from the starting point of inference when the current time point is set as the starting point. The same representations in the image D6 shown in FIG. 8 as those in the above-described image D1 to image D5 are denoted by the same reference signs, and descriptions thereof will not be repeated.

The image D6 shows a representation p3 indicating the transition of the bone density of the reference person different from the subject. In other words, the image generator 424 generates the image D6 showing the transition information indicating the transition of the bone density of the reference person different from the subject. For example, the storage 43 may store reference person data indicating the transition of the bone density of the reference person. The image generator 424 may generate a display image showing the representation p3, which is information indicating the transition of the bone density of the reference person, using reference person data stored in the storage 43.

For example, the reference person may be a person having the same attributes as the subject. Here, the same attributes may mean that at least one selected from the group consisting of age, weight, gender, disease, blood relative, type of osteoporosis, value by blood marker, and evaluation by fracture risk assessment tool (FRAX (trade name)) is equivalent or similar. The transition of the bone density of the reference person may be compared with the transition of the bone density of the subject. For example, the reference person may be a person who has already received treatment for bone density, or a person who has not received treatment for bone density.

### Example of Display Image Showing Inferred Value in Accordance with Change in Timing to Start Treatment for Subject

An example of a display image showing inferred values according to a change in the timing to start treatment for a subject will be described.

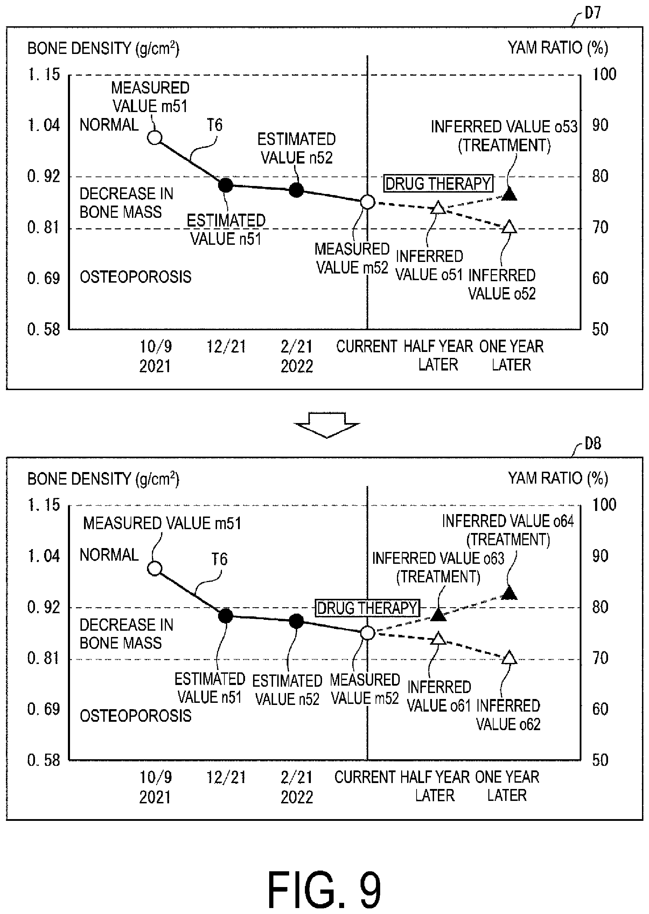

FIG. 9 is a diagram showing an example of a display image that transitions in accordance with a change in the timing to start treatment for a subject. In this example, a transition of the image when the timing to start treatment for the subject is changed from half a year later to the current time point through an operation input of the user will be described. As illustrated in FIG. 9, when a change is made, the display image shown transitions from the image D7 to the image D8. The timing to start treatment may be set to any time point through an operation input of the user.

The image D7 is an example of a display image showing inferred values of the bone density of the subject when treatment is started for the subject half a year later. The image D7 shows the measured values m51 and m52 of the bone density of the subject at the time points of the measurement, estimated values n51 and n52 of the bone density of the subject at the time points at which the medical image was captured, and a transition T6 of the bone density of the subject based on the measured values and the estimated values. The image D7 shows the inferred value o51, inferred value o52, and inferred value o53. The same representations in the image D7 as those in the above-described image D1 to image D6 are denoted by the same reference signs, and descriptions thereof will not be repeated.

The inferred value o51 is an inferred value of the bone density of the subject at a time point in half a year from the current time point set as the starting point of the inference.

The inferred value o52 is an inferred value of the bone density of the subject in one year from the current time point set as the starting point of the inference when treatment for the subject is started half a year later. For the inferred value o52, and inferred value o53, the inferred value o62 of the image D8, and the inferred value o64 of the image D8, which will be described below, the inferred values are shown at the end points of the connected dotted lines, and the starting points of the dotted lines are connected to the inferred values of the bone densities of the subject in half a year from the current time point. The starting points of the dotted lines connected to these inferred values do not indicate the time point that serves as the starting point of the inference.

The inferred value o53 is an inferred value of the bone density of the subject in one year from the current time point set as the starting point of the inference when treatment for the subject is not started.

The image D8 is an example of a display image showing inferred values of the bone density of the subject when treatment for the subject is started at the current time point. The same representations in the image D8 as those in the above-described image D1 to image D7 are denoted by the same reference signs, and descriptions thereof will not be repeated. In the image D8, the inferred values o51, o52, o63, and o64 are shown.

The inferred value o61 is an inferred value of the bone density of the subject in half a year from the current time point set as the starting point of the inference when treatment for the subject is not started.

The inferred value o62 is an inferred value of the bone density of the subject in one year from the current time point set as the starting point of the inference when treatment for the subject is not started.

The inferred value o63 is an inferred value of the bone density of the subject in half a year from the current time point set as the starting point of the inference when treatment for the subject is started at the current time point.

The inferred value o54 is an inferred value of the bone density of the subject in one year from the current time point set as the starting point of the inference when treatment for the subject is started at the current time point.

Although an example in which the image displayed in accordance with the change in the timing to start treatment transitions from the image D7 to the image D8 has been described in the present example, the image D7 and the image D8 may be displayed simultaneously.

Each of the inferred values corresponding to the different timings to start the treatment may be displayed in one image, for example, in one graph.

### Other Example

Another example of the above-described image D1 to image D8 will be described.

### Example Showing Names of Bones Captured in Medical Image Used for Estimation of Estimated Value

In the image D1 to image D8, the names of bones captured in the medical images used for estimating respective estimated values may be shown. That is, the image generator 424 may generate a display image showing information indicating the name of a bone captured in a medical image used for estimating the estimation information 433. The first generator 422 may generate the estimation information 433 from images of bones of a plurality of sites captured in a medical image. The image generator 424 may generate a display image showing information indicating the names of a plurality of bones captured in a medical image used for estimating the estimation information 433. The names of the bones may be, for example, at least one selected from the group consisting of skull, clavicle, sternum, scapula, rib, humerus, radius, ulna, hand bone, spine, sacrum, coccyx, femur, patella, tibia, fibula, foot bone, and the like.

In the image D1 to image D8, the names of bones captured in the medical image used for inferring respective inferred values may be shown. That is, the image generator 424 may generate a display image showing information indicating the names of the bones captured in a medical image used for inferring the inference information 434. The second generator 427 may generate the inference information 434 from images of bones of a plurality of sites captured in a medical image. The image generator 424 may generate a display image showing information indicating the names of the bones captured in a medical image used for inferring the inference information 434.

### Example of Displaying Medical Image Used for Estimation of Estimated Value and Inference of Inferred Value

The image D1 to image D8 may show a medical image used for estimation of each estimated value and inference of each inferred value. The medical image may a medical image obtained by capturing, for example, the head, neck, chest, lumbar region (lumbar vertebrae: L1 to L4, etc.), hip joint, knee joint, ankle joint, foot, toes, shoulder joint, elbow joint, wrist joint, hand, fingers, jaw joint, or the like. The type of site to be captured in a medical image is not limited thereto. The medical image may be a front image capturing a target site radiated with a simple X-ray from the front, or may be a side image capturing the target site from a side. An X-ray image may be an image capturing a cortical bone and/or a trabecular bone.

### Example of Display When Bone Density is Estimated or Inferred from Each of Plurality of Sites

When the bone density is estimated or inferred from each of a plurality of sites captured in a medical image, the image D1 to the image D8 may show graphs each indicating a transition of the bone density in each site. For example, the captured image of each site used for estimation of each estimated value and inference of each inferred value may be superimposed on each corresponding graph to be displayed. The plurality of sites may be set as, for example, regions for each vertebral body. The plurality of sites may be set as, for example, regions in which a plurality of vertebral bodies are collected. The plurality of sites may be set as, for example, regions having different areas. The plurality of sites may be set as regions in which the area of a specific portion of a certain site is divided. The plurality of sites may be arbitrarily set as, for example, regions depending on attribute information of the subject. For example, when the subject has a history of fractures, the area of the specific portion may be set to be smaller than that of a subject with no history of fractures.

### Example of Display of Determination Criterion

In a display image showing information indicating a determination criterion such as normal, decrease in bone mass, and osteoporosis, the determination criteria may be superimposed on corresponding graphs to be displayed. The determination criterion may be according to the age of the subject at the time of measurement, the age of the subject at the time of estimation, or the age of the subject at the time of inference. The time of inference mentioned here may be, for example, the third time point described above.

Example of Displaying Error Range of Inferred Value or Inference Probability The second generator 427 may generate the inference information 434 including information indicating an error range of an inferred value. The image generator 424 may generate a display image showing information indicating the error range for each inferred value.

In more detail, the second generator 427 may generate the inference information 434 including information indicating an inference probability of the inferred value or the error range of the inferred value. The inference probability may be, for example, a prediction probability. The inference probability and/or the error range may be shown in the above-described image D1 to image D8.

### Example of Displaying Error Range of Inferred Value

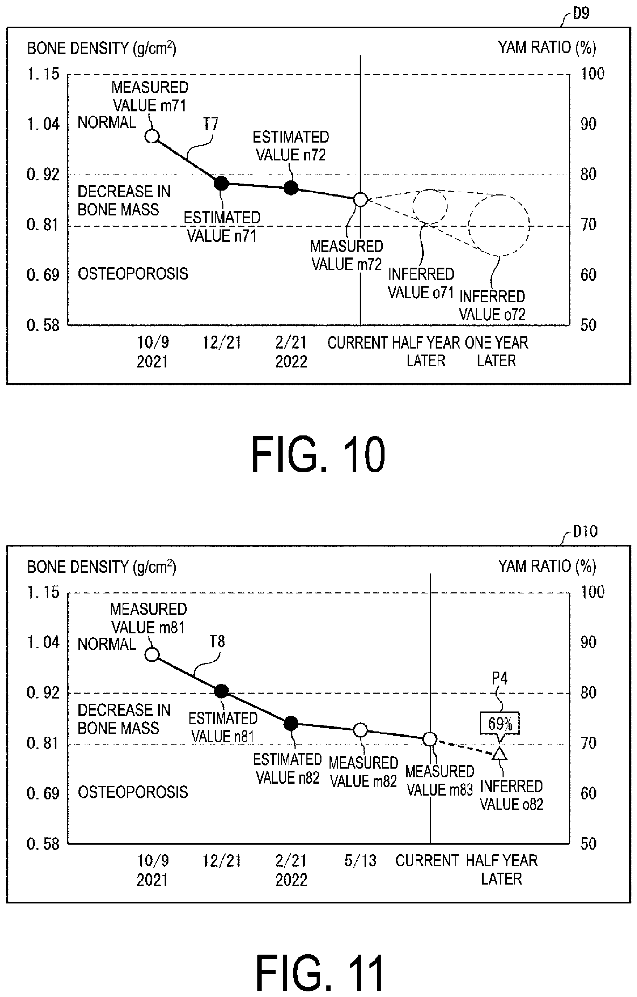

Here, an example of a display image showing an error range of an inferred value will be described. FIG. 10 is a diagram showing an example of a display image showing an error range of inferred values of a bone density of a subject. The image D9 shown in FIG. 10 shows the measured values m71 and m72 of the bone density of the subject at the time points of the measurement, estimated values n71 and n72 of the bone density of the subject at the time points at which the medical image was captured, and a transition T7 of the bone density of the subject based on the measured values and the estimated values. The inferred value o71 and inferred value o72 are shown in the image D9.

The inferred value o71 is an inferred value of the bone density of the subject in a predetermined period including a time point in half a year from the starting point of the inference when the current time point is set as the starting point. The inferred value o72 is an inferred value of the bone density of the subject in a predetermined period including a time point one year after the starting point of the inference when the current time point is set as the starting point. That is, the inferred value o71 and inferred value o72 may be shown as inferred values of the bone density of the subject in a predetermined period including a time point that comes after a predetermined period elapses from the starting point of the inference when the current time point is set as the starting point.

In the example shown in FIG. 10, the inferred value o71 and inferred value o72 are indicated by circles including the inferred values and the error ranges of the inferred values. The error ranges of the inferred value o71 and inferred value o72 may be indicated by error bars or box plots. The upper limits of the error ranges of the inferred value o71 and inferred value o72 may be connected by a dotted line or the like for indication. The lower limits of the error ranges of the inferred value o71 and inferred value o72 may be connected by a dotted line or the like for indication.

### Example in which Estimated Value is Range

The first generator 422 may generate the estimation information 433 including information indicating the width of each estimated value. The image generator 424 may generate a display image showing the width of each estimated value. As a specific example of the width of an estimated value, an estimated bone density width of 0.79 to 0.81 (median value 0.80) g/cm² or the like can be exemplified.

Example of Displaying Inference Probability of Inferred Value An example of a display image showing an inference probability of an inferred value will be described. FIG. 11 is a diagram showing an example of a display image showing an inference probability of an inferred value of a bone density of a subject. The image D10 shown in FIG. 11 shows the measured values m81 to m83 of the bone density of the subject at the time points of the measurement, estimated values n81 and n82 of the bone density of the subject at the time points at which the medical image was captured, and a transition T8 of the bone density of the subject based on the measured values and the estimated values. An inferred value o82 is shown in the image D10.

The inferred value o82 is an inferred value of the bone density of the subject at a time point in half a year from the starting point of the inference when the current time point is set as the starting point. The image D10 shows a representation p4 "69%" indicating the inference probability of the inferred value o82 above the inferred value o82. That is, the second generator 427 may generate the inference information 434 indicating the inference probability related to the inferred value. The image generator 424 may generate a display image showing the inference probability of each inferred value.

Example of Displaying Measurement Result When Measuring Measured Value The image D1 to the image D10 may further show a medical image captured when each measured value was measured. The medical image may a medical image obtained by capturing, for example, the head, neck, chest, lumbar region (lumbar vertebrae: L1 to L4, etc.), hip joint, knee joint, ankle joint, foot, toes, shoulder joint, elbow joint, wrist joint, hand, fingers, jaw joint, or the like. The type of site to be captured in a medical image is not limited thereto. The medical image may be a front image capturing a target site radiated with a simple X-ray from the front, or may be a side image capturing the target site from a side. An X-ray image may be an image capturing a cortical bone and/or a trabecular bone. The image D1 to the image D10 may show the measurement results corresponding to the indicated measured values. For example, the measurement results shown may be indication of measurement results obtained by using the DXA method.

### Another Example of Displaying Medical Image Used for Estimation of Estimated Value and Inference of Inferred Value

When the image D1 to the image D10 further show medical images used for estimation of each estimated value and inference of each inferred value, the medical images may be shown such that the regions used for the estimation are identifiable.

For example, the first generator 422 may generate a heat map for a region used for estimating an estimated value. In this case, for example, the outer edge of the heat map may indicate segmented areas in the region used for estimation. The heat map may indicate a magnitude of a bone density with a concentration of any color. For example, the first generator 422 may generate a heat map indicating the level of focus. A heat map indicating numerical values of a bone density may be generated. The first generator 422 may generate a heat map indicating the likelihood (probability) of a fracture. A display image may show an image in which a heat map is superimposed on a medical image. Images used for the heat map may be still images or moving images. By using a moving image for representation, for example, by sequentially fading various heat maps, the relationship between the heat maps can be visually recognized with ease. When an analysis result is a heat map of a bone density including areas other than the segmented areas, a part of the segmented areas may be surrounded by a frame.

### Method of Displaying Medical Image and Measurement Result

When the image D1 to the image D10 further show medical images used for estimation of each estimated value and inference of each inferred value, if a cursor of a mouse or the like is overlapped on a display area of an estimated value or a display area of an inferred value, a medical image corresponding to the estimated value or the inferred value may be shown. Here, the corresponding medical image may be a medical image used for estimating the estimated value or inferring the inferred value. For example, the medical image may be shown such that the region used for estimating the estimated value or inferring the inferred value for the medical image is identifiable. When a cursor of a mouse or the like is overlapped on the display area of a measured value, the measurement result corresponding to the measured value may be displayed. For example, the measurement results shown may be indication of measurement results obtained by using the DXA method. The medical image or the measurement results may be displayed in a balloon from the display area of the measured value or the display area of the inferred value on which a cursor of a mouse or the like is overlapped. When a click operation is performed on the display area of the estimated value, the inferred value, or the measured value, a display image including display of the medical image or the measurement result described above may be shown.

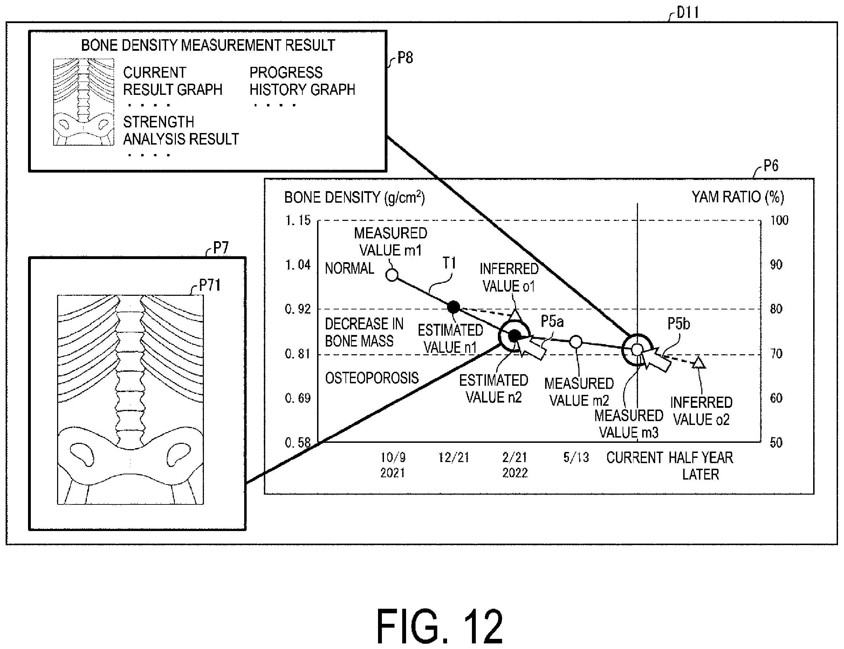

An example of a display image showing a medical image corresponding to an estimated value and a measured value will be described. FIG. 12 is a diagram illustrating an example of a display image showing a medical image used for estimation of estimated values and measurement results corresponding to measured values. The same representations in FIG. 12 as those in FIG. 3 described above are denoted by the same reference signs, and descriptions thereof will not be repeated. The image D11 illustrated in FIG. 12 includes a representation P6 indicating a graph showing a transition T1 of a bone density, a representation P7 for the medical image corresponding to the estimated value n2, and a representation P8 for the measurement result corresponding to the measured value m3. As illustrated in FIG. 12, when the cursor P5a of a mouse is overlapped on the display area of the estimated value n2, the image D11 may show the representation P7 for the medical image corresponding to the estimated value n2. For example, when the operation receiver 423 receives an operation of overlapping the cursor P5a of the mouse on the display area of the estimated value n2, the image generator 424 generates a display image including the representation P7, and the display controller 426 causes the display 45 to display the display image. Further, as illustrated in FIG. 12, when the cursor P5b of the mouse is overlapped on the display area of the measured value m3, the representation P8 for the measurement result corresponding to the measured value m3 may be displayed.

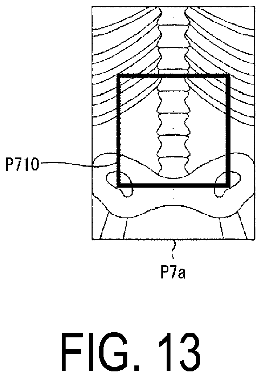

FIG. 13 is a diagram illustrating another example of display of a medical image corresponding to the estimated value n2. In this example, when the cursor P5a of the mouse is overlapped on the display area of the estimated value n2 shown in FIG. 12, a representation P7a for the medical image corresponding to the estimated value n2 is displayed. In the representation P7a, a representation P710 that is a frame line indicating a region used for estimation of the estimated value n2 in the medical image is displayed.

### Example of Displaying Transition of Bone Density of Each of Plurality of Sites

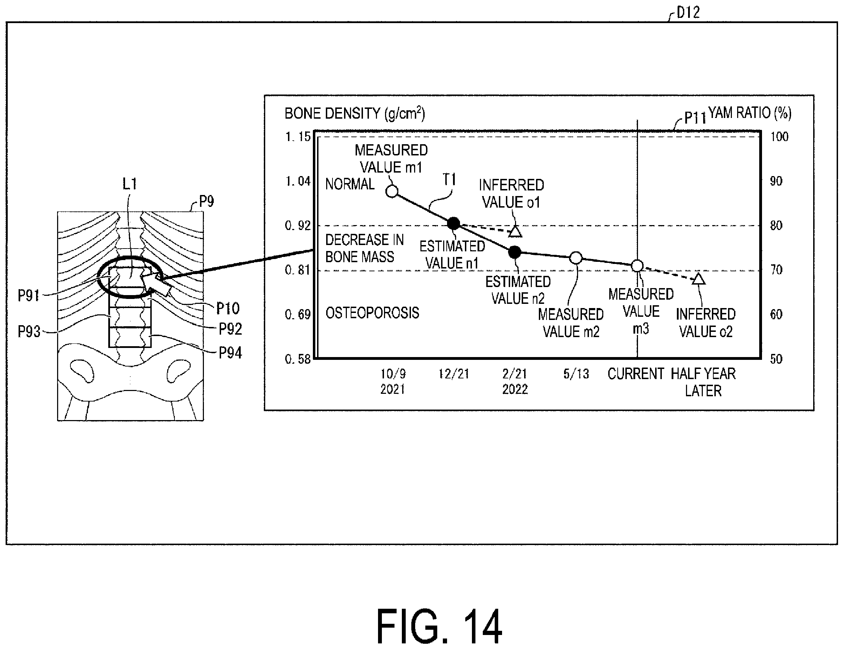

A display image displayed on the display 45 may show a transition of a bone density of each of a plurality of sites captured in a medical image. An example of the display image showing a transition of a bone density of each of a plurality of sites will be described. FIG. 14 is a diagram illustrating an example of a display image showing a transition of bone density of each of a plurality of sites. The same representations in FIG. 14 as those in FIG. 3 described above are denoted by the same reference signs, and descriptions thereof will not be repeated. The image D12 illustrated in FIG. 14 includes a representation P11 indicating a graph indicating the transition T1 of the bone density and a representation P9 for a medical image. The representation P9 of the medical image shows a representation P91 to a representation P94 with frame lines indicating the regions in which L1 to L4, which are the sites of lumbar vertebrae, were captured. For example, when the cursor P10 of the mouse is positioned within the representation P91 which is the display area of L1 lumbar vertebra, an image indicating the transition of the bone density at L1 lumbar vertebra may be shown, as illustrated in FIG. 14. A display image that simultaneously shows the transition of the bone density of each of L1 to L4 lumbar vertebrae may be shown. In such a display image, for example, when the cursor P10 of the mouse is positioned within the representation P91 which is the display area of L1 lumbar vertebra, the transition of the bone density of L1 lumbar vertebra may be highlighted for display.

Example of Displaying Inferred Fracture Location as Inference Result The operation receiver 423 may receive an operation input to instruct inference for a fracture of a subject. Upon receiving a corresponding operation input, the operation receiver 423 instructs the second generator 427 to generate the inference information 434 indicating inference for a fracture of the subject. Further, the operation receiver 423 instructs the image generator 424 to generate a display image showing the inference for a fracture of the subject indicated by the inference information 434. The operation receiver 423 receives an operation input to instruct display termination. Upon receiving the operation input, the operation receiver 423 instructs the display controller 426 to terminate the display.

### Another Example of Second Generator 427

The second generator 427 included in the controller 42 may perform the following processing. The second generator 427 generates the inference information 434 including information on the possibility of a fracture of the subject occurring in the future, the fracture being inferred from the medical image indicated by the medical image data 432 stored in the storage 43. For example, the second generator 427 may generate the inference information 434 including information on the possibility of a fracture occurring in the future, the fracture being inferred from a medical image capturing the bone of the subject at the first time point. The inference information 434 may include, for example, at least one selected from the group consisting of the possibility of a fracture of the subject occurring in the future, a time inferred for the occurrence of a fracture, information indicating the location that is vulnerable to a fracture, and the like.

The second generator 427 may generate the inference information 434 including information on a fracture of the subject inferred from the medical image capturing the bone of the subject at the second time point. The inference information 434 may include information on a fracture of the subject at a fourth time point. The fourth time point may be a time point different from the second time point and the third time point.

When the image generator 424 generates an image using the inference information 434, the image generator 424 may generate a display image showing the inference information 434 associated with the fourth time point.

### Another Example of Inference Model 4271

The inference model 4271 included in the second generator 427 according to the present example may perform the following processing. The inference model 4271 may infer, from a medical image indicated by the medical image data 432 of the subject, information on a possibility of a fracture of the subject occurring in the future at a time point different from the time point at which the medical image was captured. The inference model 4271 may be set based on learning data including a medical image (fourth image) showing a bone of a third person and training data including information on a fracture of the third person. The "third person" mentioned above may not be particularly limited to humans. For example, it may be an animal of the same species as the "subject".

For example, information on a fracture of a person having a bone captured in a simple X-ray image indicated by corresponding learning image data may be associated, as training data, with each of a plurality of pieces of learning image data included in learning data. The training data may be bone evaluation information and/or a medical image (fifth image) showing a fractured location of the third person is captured, and the like. The fifth image may be an image capturing the same site as that in the fourth image. The fifth image may be an image capturing a site different from that in the fourth image.

The training data may be measured at a time different from the time when the image data for learning was captured. The training data may be measured at the same time as the time when the image data for learning was captured.

The inference model 4271 may be composed of a plurality of models. For example, the inference model 4271 may be composed of a model for inferring, from a medical image, a bone density of a subject at a time point different from the time point at which the medical image was captured, a model for inferring, from a medical image, a possibility of a fracture of a subject occurring in the future at a time point different from the time point at which the medical image was captured, and the like. The inference results output from the plurality of models may be displayed together or only one of the inference results may be displayed in a display image displayed on the display 45.

The inference information 434 may be a method for indicating information of a different type from the first method and the second method. For example, the first method may be for indicating information on the bone density, and the second method may be for indicating information on a fracture. For example, a measured value of the bone density of the subject and an estimated value of the bone density of the subject may be shown in the same display mode in a display image displayed on the display 45. The information on a fracture of the subject may be shown in a display image displayed on the display 45 in a display mode different from the display mode of the measured value of the bone density of the subject and the estimated value of the bone density of the subject.

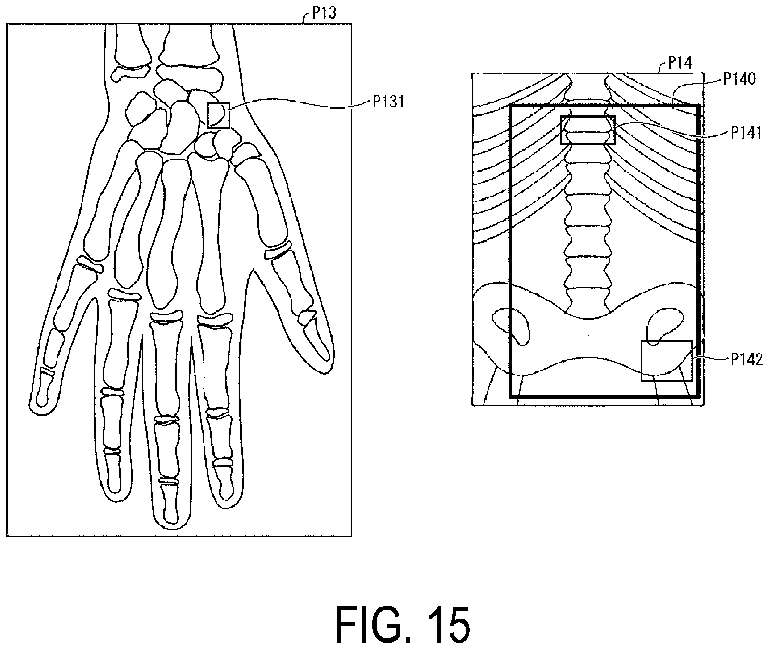

The display image displayed on the display 45 may show a portion having a high possibility of a fracture occurring in the future indicated by the inference information 434. FIG. 15 is a set of diagrams illustrating an example of display of a portion having a high possibility of a fracture, included in a display image displayed on the display 45. In the example illustrated in the left diagram of FIG. 15, the display image displayed on the display 45 includes a representation P13 that is a representation of an X-ray image. The representation P13 includes a representation P131 that is a frame line indicating a region with a high possibility of a fracture occurring in the future.

In the example illustrated in the right diagram of FIG. 15, the display image displayed on the display 45 includes a representation P14 that is a representation of an X-ray image. The representation P14 includes a representation P14 that is a frame line indicating a region used to infer the bone density, and a representation P141 and a representation P142 that are frames indicating regions with a high possibility of a fracture occurring in the future. For example, an indication such as "inference" may be displayed near the representation P141 and the representation P142 that are frame lines indicating the region with a high possibility of a fracture occurring in the future.

The display apparatus 4 may include a user interface that receives a user operation for switching between display and non-display of inferred values in a display image. The interface may receive a user operation for selecting an inferred value of the time point to be displayed. The interface may receive a user operation to switch between display and non-display of medication, nutritional guidance, and exercise guidance in the display image.

### Second Embodiment

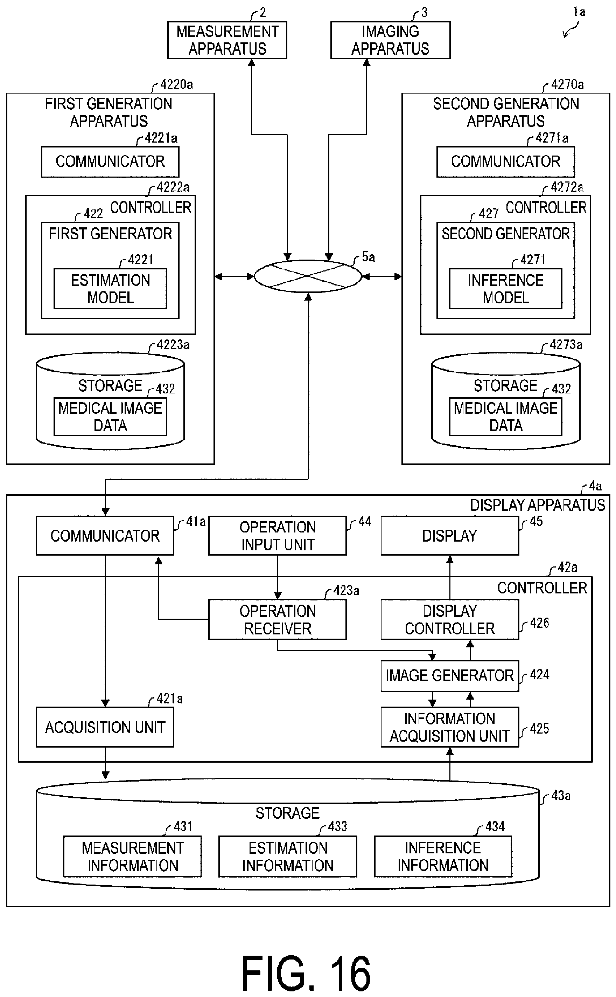

Another embodiment of the present disclosure will be described below with reference to FIG. 16. For convenience of description, members having the same functions as those of the members described in the above-described embodiment are denoted by the same reference signs, and description thereof is not repeated.

FIG. 16 is a block diagram illustrating a main part configuration of an example of an information processing system 1a according to the present embodiment. As illustrated in FIG. 16, the information processing system 1a includes a measurement apparatus 2, an imaging apparatus 3, a display apparatus (image generation apparatus) 4a, a first generation apparatus 4220a, and a second generation apparatus 4270a. Each of the apparatuses performs wired or wireless communication. Further, the communication may be performed via a network 5a as illustrated in FIG. 16.

The first generation apparatus 4220a performs processing identical or similar to the processing performed by the first generator 422 described in the first embodiment. The first generation apparatus 4220a transmits the generated estimation information 433 to the display apparatus 4 via a communicator 4221a.

The second generation apparatus 4270a performs processing identical or similar to the processing performed by the second generator 427 described in the first embodiment. The second generation apparatus 4270a receives an instruction to generate the inference information 434 via a communicator 4271a. The second generation apparatus 4270a transmits the generated inference information 434 to the display apparatus 4 via the communicator 4271a.

The display apparatus 4a includes a communicator 41a, a controller 42a, a storage 43a, an operation input unit 44, and a display 45. An acquisition unit 421a of the controller 42a receives the measurement information 431, the estimation information 433, and the inference information 434 via the communicator 41a. The display apparatus 4a performs processing identical or similar to the image generation process described in the first embodiment. When an operation receiver 423a receives an instruction to receive an operation input instructing inference for a bone of the subject, the display apparatus 4a transmits an instruction to generate the inference information 434 to the second generation apparatus 4270a via the communicator 41a. The operation receiver 423a receives an operation identical or similar to that of the operation receiver 423. Further, the display apparatus 4a that functions as an operation input apparatus that performs processing of the operation input unit 44 and the operation receiver 423a may be included in the information processing system 1a as a separate apparatus. In this case, the operation input apparatus may communicate with the display apparatus 4a, the second generation apparatus 4270a, and the like via the network 5a.

### Third Embodiment

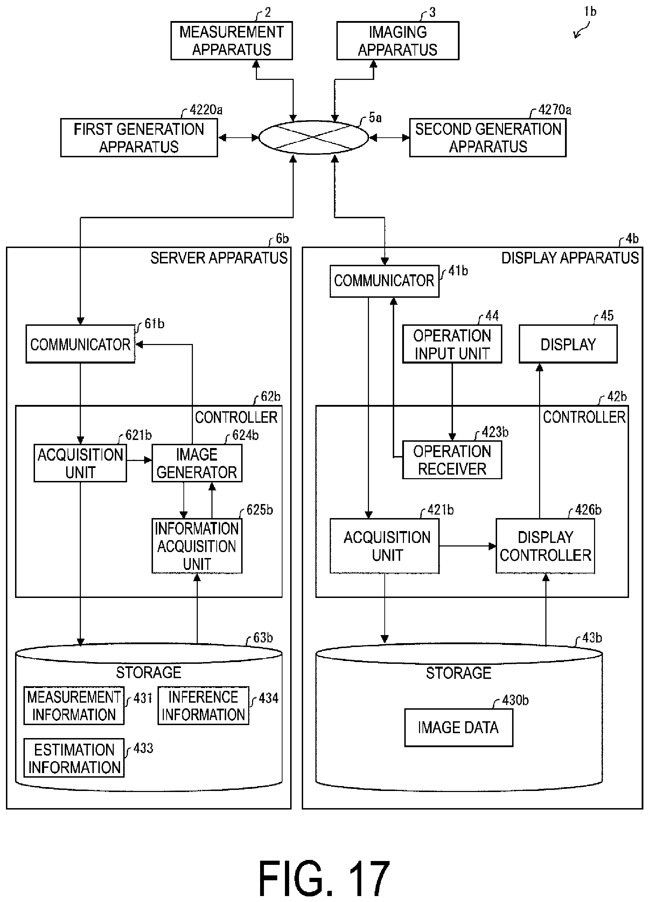

Another embodiment of the present disclosure will be described below with reference to FIG. 17. For convenience of description, members having the same functions as those of the members described in the above-described embodiment are denoted by the same reference signs, and description thereof is not repeated.

FIG. 17 is a block diagram illustrating a main part configuration of an example of an information processing system 1b according to the present embodiment. As illustrated in FIG. 17, the information processing system 1b includes a measurement apparatus 2, an imaging apparatus 3, a display apparatus 4b, a first generation apparatus 4220a, a second generation apparatus 4270a, and a server apparatus (image generation apparatus) 6b. Each of the apparatuses performs wired or wireless communication. Further, the communication may be performed via a network 5a as illustrated in FIG. 17. The server apparatus 6b may be a cloud server.

The first generation apparatus 4220a performs processing identical or similar to the processing performed by the first generator 422 described in the first embodiment. The first generation apparatus 4220a transmits the generated estimation information 433 to the server apparatus 6b via a communicator 4221a.

The second generation apparatus 4270a performs processing identical or similar to the processing performed by the second generator 427 described in the first embodiment. The second generation apparatus 4270a receives an instruction to generate the inference information 434 via a communicator 4271a. The second generation apparatus 4270a transmits the generated inference information 434 to the server apparatus 6b via the communicator 4271a.

The server apparatus 6b includes a communicator 61b, a controller 62b, and a storage 63b. An acquisition unit 621b of the controller 62b stores the measurement information 431, the estimation information 433, and the inference information 434 received via the communicator 61b in the storage 63b. An information acquisition unit 625b and an image generator 624b of the controller 62b perform an image generation process identically or similarly to the information acquisition unit 425 and the image generator 424 described in the first embodiment. The server apparatus 6b transmits generated image data 430b to the display apparatus 4b via the communicator 61b.

The display apparatus 4b includes a communicator 41b, a controller 42b, a storage 43b, an operation input unit 44, and a display 45. When an operation receiver 423b receives an instruction to receive an operation input instructing inference for a bone of the subject, the display apparatus 4b transmits an instruction to generate the inference information 434 to the second generation apparatus 4270a via the communicator 41b. The operation receiver 423b receives an operation identical or similar to that of the operation receiver 423. An acquisition unit 421b of the controller 42b acquires image data 430b from the server apparatus 6b via the communicator 41b. The acquisition unit 421b stores the acquired image data 430b in the storage 43b, and transmits a signal indicating that the image data 430b has been stored to a display controller 426b. Upon receiving the signal from the acquisition unit 421b, the display controller 426b acquires the image data 430b from the storage 43b, and displays the image represented by the image data 430b on the display 45.

### Fourth Embodiment

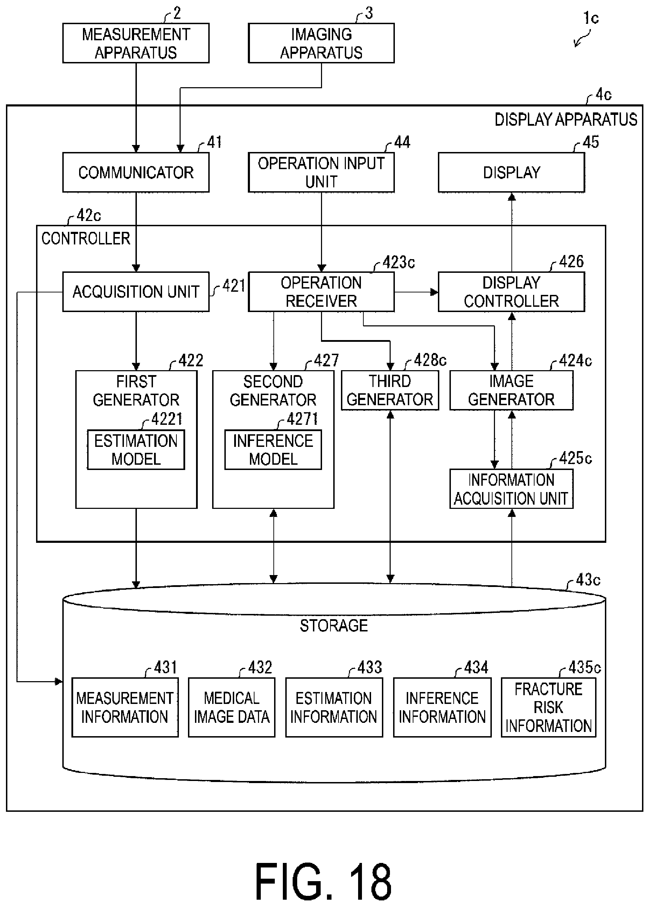

Another embodiment of the present disclosure will be described below with reference to FIG. 18. For convenience of description, members having the same functions as those of the members described in the above-described embodiment are denoted by the same reference signs, and description thereof is not repeated.

FIG. 18 is a block diagram illustrating a main part configuration of an example of an information processing system 1c according to the present embodiment. As illustrated in FIG. 18, the information processing system 1c includes a measurement apparatus 2, an imaging apparatus 3, and a display apparatus 4c. In the following, only the functions of the display apparatus 4c that are added to the display apparatus 4 described in the first embodiment will be described. The description of the same functions of the display apparatus 4c as those of the display apparatus 4 will not be repeated.

The display apparatus 4c includes a communicator 41, a controller 42c, a storage 43c, an operation input unit 44, and a display 45.

### Controller 42c

The controller 42c comprehensively controls each unit of the display apparatus 4. The controller 42c includes an acquisition unit 421 (first acquisition unit), a first generator 422, an operation receiver 423c, an image generator (generator) 424c, an information acquisition unit (first acquisition unit, second acquisition unit, third acquisition unit, and fourth acquisition unit) 425c, a display controller 426, a second generator 427, and a third generator 428c.

The operation receiver 423c, the image generator 424c, and the information acquisition unit 425c perform the following processing in addition to the processing performed by the operation receiver 423, the image generator 424, and the information acquisition unit 425.

### Operation Receiver 423c

The operation receiver 423c receives an operation input to instruct display of a display image showing third information on a bone different from first information and/or second information of the subject. Here, the third information may be information on a bone, which is calculated based on the first information and/or the second information. The third information may be information on a bone different from the first information and/or the second information, which is calculated from the measurement data and/or a medical image of the subject used to acquire the first information and/or the second information. For example, when the first information and/or the second information are bone densities of the subject, the third information may be fracture risk information of the subject. Although a case in which the first information and the second information are bone densities of the subject and the third information is fracture risk information will be described in the present embodiment, the present disclosure is not limited thereto. Upon receiving an operation input, the operation receiver 423c instructs the third generator 428c to generate fracture risk information (third information) 435c indicating a fracture risk of the subject. Further, the operation receiver 423c instructs the image generator 424c to generate a display image showing the fracture risk information 435c.

### Third Generator 428c

The third generator 428c generates the fracture risk information 435c in response to an instruction to generate the fracture risk information 435c indicating a fracture risk of the subject received from the operation receiver 423c. The third generator 428c stores the generated fracture risk information 435c in the storage 43c. For example, the third generator 428c may generate the fracture risk information 435c as follows.

The third generator 428c generates fracture risk information 435c indicating a fracture risk of the subject at a first time point from the measurement information 431 indicating the measured value of the bone density of the subject measured at the first time point.

The third generator 428c generates the fracture risk information 435c indicating a fracture risk of the subject at a second time point from the estimation information 433 indicating the estimated value of the bone density estimated from a medical image capturing the bone of the subject at the second time point.

The third generator 428c generates the fracture risk information 435c indicating a fracture risk of the subject at a third time point from the inference information 434 indicating the inferred value of the bone density of the subject at the third time point.

That is, the third generator 428c generates the fracture risk information 435c on the bone of the subject calculated from at least one selected from the group consisting of the measurement information 431, the estimation information 433, and the inference information 434.

The third generator 428c may calculate a fracture risk from at least one selected from the group consisting of a measured value, an estimated value, and an inferred value of the bone density by using a fracture risk evaluation tool. For the fracture risk evaluation tool, for example, FRAX (trade name) may be used.

On the other hand, the third generator 438c may generate the fracture risk information 435c of the subject at the first time point from the measurement data related to the bone of the subject at the first time point measured by the measurement apparatus 2. The third generator 428c may generate the fracture risk information 435c of the subject at the second time point from a medical image capturing the bone of the subject at the second time point. The third generator 428c may generate the fracture risk information 435c of the subject at the third time point from a medical image capturing the bone of the subject at the second time point.

That is, the third generator 428c may generate the measurement data on the bone of the subject at the first time point measured by the measurement apparatus 2, or the fracture risk information 435c on the bone of the subject at the second time point, and the fracture risk information 435c on the bone of the subject calculated from at least one of the medical images showing the bone of the subject at the second time point.

### Image Generator 424c

Upon receiving an instruction to generate a display image showing the fracture risk information 435c, the image generator 424c acquires the measurement information 431, the estimation information 433, and the fracture risk information 435c in the storage 43c through the information acquisition unit 425c. The image generator 424c generates a display image showing the fracture risk information 435c of the subject by using the fracture risk information 435c. The image generator 424c transmits data of the generated display image to the display controller 426. An example of the display image generated by the image generator 424c will be described in detail below.

In the present embodiment, an example in which the third generator 428c calculates the fracture risk from a value of bone density will be described. As another example, the third generator 428c may calculate the YAM (%), T-score, or Z-score from the value of bone density indicated by at least one selected from the group consisting of the measurement information 431, the estimation information 433, and the inference information 434. That is, the third generator 428c may generate information indicating the YAM (%), T-score, or Z-score. The image generator 424c may generate a display image showing the YAM (%), T-score, or Z-score calculated from at least one selected from the group consisting of the measurement information 431, the estimation information 433, and the inference information 434.

### Example of Displaying Fracture Risk of Subject

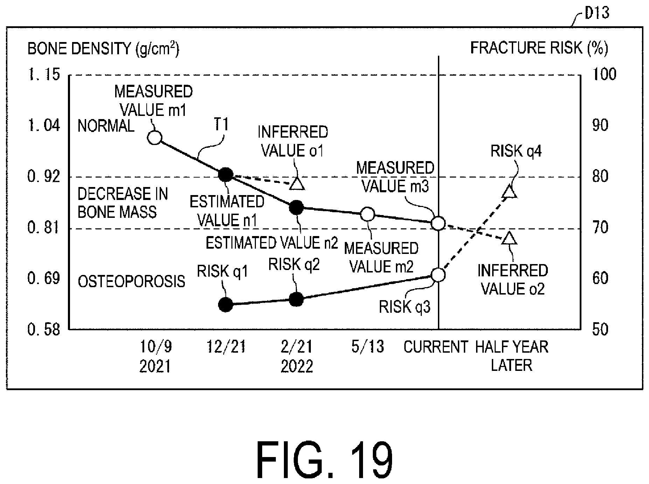

An example of a display image showing a fracture risk of the subject will be described with reference to FIG. 19. FIG. 19 is a diagram illustrating an example of a display image showing a fracture risk of a subject. The image D13 shown in FIG. 19 shows measured values m1 to m3 of the bone density at the time points of the measurement, estimated values n1 and n2 of the bone density of the subject at the time point at which the medical image was captured, and a transition T1 of the bone density based on the measured values and the estimated values. The inferred value o1 and inferred value o2 are shown in the image 13. The same representations in the image D13 as those in the above-described image D1 are denoted by the same reference signs, and descriptions thereof will not be repeated.

The image D13 shows a fracture risk q1, a fracture risk q2, a fracture risk q3, and a fracture risk q4, which indicate the risk of fractures. The fracture risk q1 is a value indicating the fracture risk calculated from the estimated value n1. The fracture risk q2 is a value indicating the fracture risk calculated from the estimated value n2. The fracture risk q3 is a value indicating the fracture risk calculated from the measured value m3. The fracture risk q4 is a value indicating the fracture risk calculated from the inferred value o2.

The fracture risk calculated from the measured value, the fracture risk calculated from the estimated values, and the fracture risk calculated from the inferred value may be displayed in different display modes. In the example shown in FIG. 19, the fracture risk q1 and the fracture risk q2 calculated from the estimated values are indicated by filled circles, the fracture risk q3 calculated from the measured value is indicated by a white circle, and the fracture risk q4 calculated from the inferred value is indicated by a white triangle.

### Fifth Embodiment

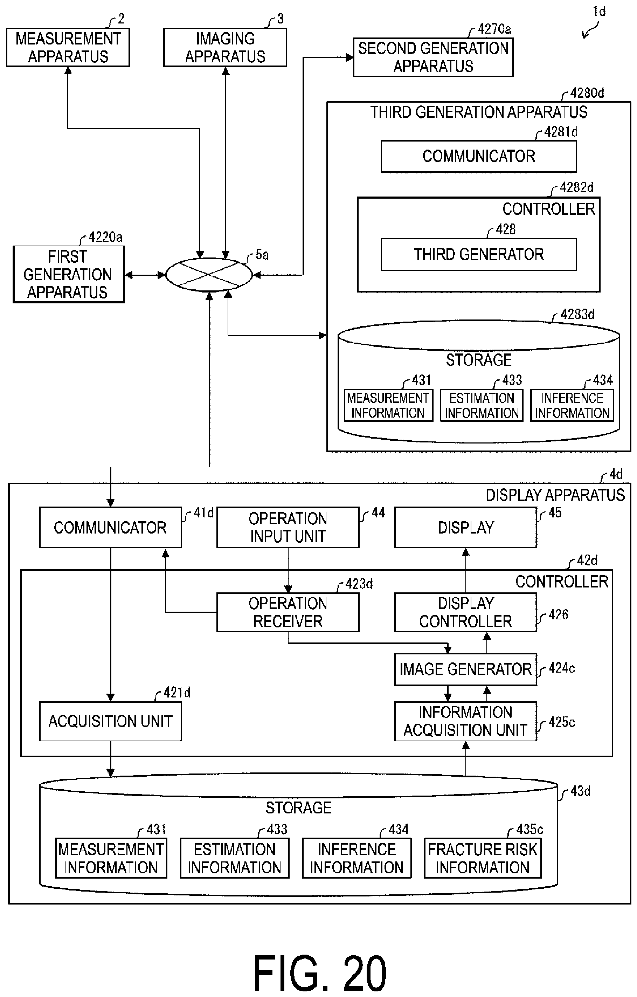

Another embodiment of the present disclosure will be described below with reference to FIG. 20. For convenience of description, members having the same functions as those of the members described in the above-described embodiment are denoted by the same reference signs, and description thereof is not repeated.

FIG. 20 is a block diagram illustrating a main part configuration of an example of an information processing system 1d according to the present embodiment. As illustrated in FIG. 20, the information processing system 1d includes a measurement apparatus 2, an imaging apparatus 3, a display apparatus (image generation apparatus) 4d, a first generation apparatus 4220a, a second generation apparatus 4270a, and a third generation apparatus 4280d. Each of the apparatuses performs wired or wireless communication. Further, the communication may be performed via a network 5a as illustrated in FIG. 20. The processing performed by each apparatus included in the information processing system 1d described below may be performed by another apparatus included in the information processing system 1d. That is, localization of the functions executed by each apparatus included in the information processing system 1d is not limited.

The first generation apparatus 4220a performs processing identical or similar to the processing performed by the first generation apparatus 4220a described in the second embodiment.

The second generation apparatus 4270a performs processing identical or similar to the processing performed by the second generation apparatus 4270a described in the second embodiment. The second generation apparatus 4270a receives an instruction to generate the inference information 434 via a communicator 4271a. The second generation apparatus 4270a transmits the generated inference information 434 to the display apparatus 4d via the communicator 4271a.

The third generation apparatus 4280d performs processing identical or similar to the processing performed by the third generator 428c described in the fourth embodiment. A storage 4283d included in the third generation apparatus 4280d stores measurement information 431, estimation information 433, and inference information 434. For example, the third generation apparatus 4280d acquires these pieces of information via the network 5a.

The third generation apparatus 4280d receives an instruction to generate fracture risk information 435c via the communicator 4281d. The third generation apparatus 4280d transmits the generated fracture risk information 435c to the display apparatus 4d via the communicator 4281d.

The display apparatus 4d includes a communicator 41d, a controller 42d, a storage 43d, an operation input unit 44, and a display 45. An acquisition unit 421d of the controller 42d receives the measurement information 431, the estimation information 433, the inference information 434, and fracture risk information 435c via the communicator 41d. The display apparatus 4d performs processing identical or similar to the image generation process described in the fourth embodiment. When an operation receiver 423d receives an operation input to instruct display of a fracture risk of the subject, the display apparatus 4d transmits an instruction to generate the fracture risk information 435c to the third generation apparatus 4280d via the communicator 41d. The operation receiver 423d receives an operation received by the operation receiver 423c. Further, an operation input apparatus and the display apparatus 4d that perform processing of the operation input unit 44 and the operation receiver 423c may be included in the information processing system 1d as a separate apparatus. In this case, the operation input apparatus may communicate with the display apparatus 4d, the second generation apparatus 4270a, the third generation apparatus 4280d, and the like via the network 5a.

### Sixth Embodiment

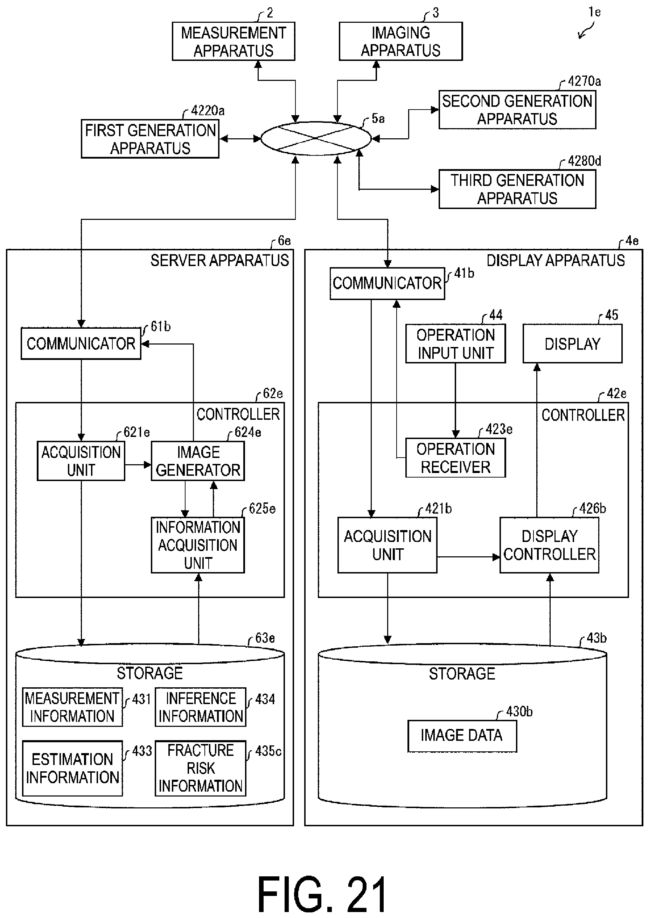

Another embodiment of the present disclosure will be described below with reference to FIG. 21. For convenience of description, members having the same functions as those of the members described in the above-described embodiment are denoted by the same reference signs, and description thereof is not repeated.

FIG. 21 is a block diagram illustrating a main part configuration of an example of an information processing system 1e according to the present embodiment. As illustrated in FIG. 21, the information processing system 1e includes a measurement apparatus 2, an imaging apparatus 3, a display apparatus 4e, a first generation apparatus 4220a, a second generation apparatus 4270a, a third generation apparatus 4270d, and a server apparatus (image generation apparatus) 6e. Each of the apparatuses performs wired or wireless communication. Further, the communication may be performed via a network 5a as illustrated in FIG. 21. The server apparatus 6e may be a cloud server. The processing performed by each apparatus included in the information processing system 1e described below may be performed by another apparatus included in the information processing system 1e. That is, localization of the functions executed by each apparatus included in the information processing system 1e is not limited.

The first generation apparatus 4220a performs processing identical or similar to the processing performed by the first generator 422 described in the first embodiment. The first generation apparatus 4220a transmits the generated estimation information 433 to the server apparatus 6e via a communicator 4221a.

The second generation apparatus 4270a performs processing identical or similar to the processing performed by the second generator 427 described in the first embodiment. The second generation apparatus 4270a receives an instruction to generate the inference information 434 via a communicator 4271a. The second generation apparatus 4270a transmits the generated inference information 434 to the server apparatus 6e via the communicator 4271a.

The third generation apparatus 4280d performs processing identical or similar to the processing performed by the third generator 428c described in the fourth embodiment. The third generation apparatus 4280d receives an instruction to generate fracture risk information 435c via the communicator 4281d. The third generation apparatus 4280d transmits the generated fracture risk information 435c to the server apparatus 6e via the communicator 4281d.

The server apparatus 6e includes a communicator 61b, a controller 62e, and a storage 63e. An acquisition unit 621e of the controller 62e stores the measurement information 431, the estimation information 433, the inference information 434, and the fracture risk information 435c received via the communicator 61b in the storage 63e. An information acquisition unit (fourth acquisition unit) 625e and an image generator 624e of the controller 62e perform processing identical or similar to the image generation process performed by the information acquisition unit 425c and the image generator 424c described in the fourth embodiment. The server apparatus 6e transmits generated image data 430b to the display apparatus 4e via the communicator 61b.

The display apparatus 4e performs the following processing in addition to the processing performed by the display apparatus 4b. The display apparatus 4e includes a communicator 41b, a controller 42e, a storage 43b, an operation input unit 44, and a display 45. When an operation receiver 423e receives an operation input to instruct display of a fracture risk of the subject, the display apparatus 4e transmits an instruction to generate the fracture risk information 435c to the third generation apparatus 4280d via the communicator 41b. The display apparatus 4e instructs the server apparatus 6e to generate display image data 430b for displaying a fracture risk of the subject. An operation receiver 423e receives an operation received by the operation receiver 423. An acquisition unit 421b of the controller 42e acquires image data 430b from the server apparatus 6e via the communicator 41b. The acquisition unit 421b stores the acquired image data 430b in the storage 43b, and transmits a signal indicating that the image data 430b has been stored to a display controller 426b. Upon receiving the signal from the acquisition unit 421b, the display controller 426b acquires the image data 430b from the storage 43b, and displays the image represented by the image data 430b on the display 45.

### Example of Implementation Using Software

The functions of the display apparatus (4, 4a, 4b, 4c, 4d, and 4e), the first generation apparatus (4220a), the second generation apparatus (4270a), the third generation apparatus (4280d), and the server apparatus (6b and 6e) (hereinafter, referred to as "apparatuses") can be implemented by a program for causing a computer to function as the apparatuses, the program for causing the computer to function as each control block (particularly, each unit included in the controller) of the apparatuses.

In such a case, the device includes a computer including at least one control device (for example, a processor) and at least one storage device (for example, a memory) as hardware for executing the above program. By executing the program by the control device and the storage device, the functions described in the embodiments are implemented.

The program may be recorded on one or more computer-readable non-transitory recording media. The recording media may be or may not be included in the device. In the latter case, the program may be supplied to the device via any wired or wireless transmission medium.

Some or all of the functions of the control blocks can be implemented by logic circuits. For example, an integrated circuit in which logic circuits functioning as the control blocks are formed is also included in the scope of the present disclosure. In addition to this, for example, a quantum computer can implement the functions of the control blocks.

The processing described in the embodiments may be executed by artificial intelligence (AI). In such a case, the AI may operate on the control device or may operate on another device (such as an edge computer or a cloud server).

The invention according to the present disclosure has been described above based on various drawings and examples. However, the invention according to the present disclosure is not limited to each embodiment described above. That is, the embodiments of the invention according to the present disclosure can be varied in various ways within the scope illustrated in the present disclosure, and embodiments obtained by appropriately combining the technical means disclosed in different embodiments are also included in the technical scope of the invention according to the present disclosure. In other words, a person skilled in the art can easily make various variations or corrections based on the present disclosure. Note that these variations or corrections are included within the scope of the present disclosure.

### Conclusion

In a first aspect of the present disclosure, an information processing system includes a first acquisition unit that acquires first information indicating information on a bone of a subject obtained based on a first method, a second acquisition unit that acquires second information indicating information on the bone of the subject obtained based on a second method that is different from the first method for the subject, and a generator that generates a display image showing the first information and the second information.

In a second aspect of the present disclosure, in the information processing system, according to the first aspect, the first information and the second information may be information on at least one selected from the group consisting of a bone density, a bone mass, and a bone quality of the subject.

In a third aspect of the present disclosure, in the information processing system according to the second aspect, the first acquisition unit may acquire the first information measured at a first time point, the second acquisition unit may acquire the second information indicating at least one selected from the group consisting of a bone density, a bone mass, and a bone quality estimated by using an estimation model from a first image capturing the bone of the subject at a second time point that is different from the first time point, and the generator may generate the display image in which the first information and the second information are arranged in chronological order of the first time point and the second time point.

In a fourth aspect of the present disclosure, in the information processing system according to the third aspect, the estimation model may be set based on learning data including a second image capturing a bone of a first person and training data including at least one selected from the group consisting of a bone density, a bone mass, and a bone quality of the first person.

In a fifth aspect of the present disclosure, the information processing system according to the third or fourth aspect may further include a third acquisition unit that acquires inference information on at least one selected from the group consisting of a bone density, a bone mass, and a bone quality inferred by using an inference model from an image capturing the bone of the subject at the first time point or the second time point, in which, if the image capturing the bone of the subject is an image capturing the bone of the subject at the first time point, the inference information may be information at a third time point that is different from the first time point, and if the image capturing the bone of the subject is an image capturing the bone of the subject at the second time point, the inference information may be information at the third time point that is different from the second time point, and the generator may generate the display image showing the inference information associated with the third time point.

In a sixth aspect of the present disclosure, in the information processing system according to the fifth aspect, the inference model may be set based on learning data including a third image capturing a bone of a second person and training data including at least one selected from the group consisting of a bone density, a bone mass, and a bone quality of the second person.

In a seventh aspect of the present disclosure, in the information processing system according to the sixth aspect, the inference information may be information on the bone density of the subject when the subject has received treatment for at least one selected from the group consisting of a bone density, a bone mass, and a bone quality, the second person may be a person who has received treatment for at least one selected from the group consisting of a bone density, a bone mass, and a bone quality, and the inference model may be set based on learning data including the third image capturing the bone of the second person who has received the treatment and training data including at least one selected from the group consisting of the bone density, the bone mass, and the bone quality of the second person.

In an eighth aspect of the present disclosure, in the information processing system according to any one of the fifth to seventh aspects, if the image capturing the bone of the subject is an image capturing the bone of the subject at the first time point, the third time point may be a time point before the first time point or a time point after the first time point, and if the image capturing the bone of the subject is an image capturing the bone of the subject at the second time point, the third time point may be a time point before the second time point or a time point after the second time point.

In a ninth aspect of the present disclosure, the information processing system according to any one of the fifth to eighth aspects may further include a fourth acquisition unit that acquires third information on the bone of the subject calculated from at least one selected from the group consisting of the first information, the second information, and the inference information, in which the generator may generate the display image showing the third information.

In a tenth aspect of the present disclosure, in the information processing system according to any one of the third to ninth aspects, the generator may generate the display image showing information indicating the name of a bone captured in the first image used for estimating the second information.

In an eleventh aspect of the present disclosure, in the information processing system according to any one of the first to tenth aspects, the generator may generate the display image showing the second information in a display mode that is different from a display mode of the first information.

In a twelfth aspect of the present disclosure, in the information processing system according to any one of the first to eleventh aspects, the generator may generate the display image showing information indicating a period of treatment for at least one selected from the group consisting of the bone density, the bone mass, and the bone quality of the subject.

In a thirteenth aspect of the present disclosure, in the information processing system according to any one of the first to twelfth aspects, the generator may generate the display image showing transition information indicating a transition of at least one selected from the group consisting of a bone density, a bone mass, and a bone quality of a reference person who is different from the subject.

In a fourteenth aspect of the present disclosure, in the information processing system according to any one of the first to thirteenth aspects, the generator may generate the display image showing information indicating a determination criterion for at least one selected from the group consisting of a bone density, a bone mass, and a bone quality.

In a fifteenth aspect of the present disclosure, in the information processing system according to any one of the first to fourteenth aspects, the generator may generate the display image including display indicating the first method and the second method.

In a sixteenth aspect of the present disclosure, the information processing system according to any one of the first to fifteenth aspects may further include a measurement apparatus that performs measurement on a bone, in which the first information may include information measured using the measurement apparatus that performs measurement on a bone.

In a seventeenth aspect of the present disclosure, the information processing system according to any one of the third to tenth aspects may further include an imaging apparatus that captures an image of a bone, in which the first image may be an image taken using the imaging apparatus.

In an eighteenth aspect of the present disclosure, an information processing method includes acquiring first information indicating information on a bone of a subject obtained based on a first method, acquiring second information indicating information on the bone of the subject obtained based on a second method that is different from the first method for the subject, and generating a display image showing the first information and the second information.

In a nineteenth aspect of the present disclosure, an image generation apparatus includes a first acquisition unit that acquires first information indicating information on a bone of a subject obtained based on a first method, a second acquisition unit that acquires second information indicating information on the bone of the subject obtained based on a second method that is different from the first method for the subject, and a generator that generates a display image showing the first information and the second information.

In a twentieth aspect of the present disclosure, an image generation program is an image generation program for causing a computer to function as the information processing system described in the nineteenth aspect, the image generation program causing the computer to function as the first acquisition unit, the second acquisition unit, and the generator.

### REFERENCE SIGNS

1, 1a, 1b, 1c, 1d, 1e Information processing system
4, 4a, 4c, 4d Display apparatus (image generation apparatus)
6b, 6e Server apparatus (image generation apparatus)
421, 421a, 421c, 621b Acquisition unit (first acquisition unit)
424, 424c, 624b, 624e Image generator (generator)
425, 425c, 625b, 625e Information acquisition unit (first acquisition unit, second acquisition unit, third acquisition unit, fourth acquisition unit)
431 Measurement information (first information)
433 Estimation information (second information)
434 Inference information
435c Fracture risk information (third information)
4221 Estimation model
4271 Inference Model
S2 First acquisition step
S3 Second acquisition step
S4 Generation step

## Claims

1. An information processing system comprising:
a first acquisition unit configured to acquire first information indicating information on a bone of a subject obtained based on a first method; and
a generator configured to generate a display image showing the first information together with information on the first method.

2. The information processing system according to claim 1,
wherein the information on the first method is related to estimation information on at least one selected from the group consisting of a bone density, a bone mass, and a bone quality estimated by using an estimation model from an image capturing the bone of the subject at a certain time point or inference information on at least one selected from the group consisting of a bone density, a bone mass, and a bone quality inferred by using an inference model from an image capturing the bone of the subject at a certain time point.

3. The information processing system according to claim 1, further comprising a second acquisition unit configured to acquire second information indicating information on the bone of the subject obtained based on a second method that is different from the first method for the subject,
wherein the generator displays the first information and the second information.

4. The information processing system according to claim 3, wherein the first information and the second information are information on at least one selected from the group consisting of a bone density, a bone mass, and a bone quality of the subject.

5. The information processing system according to claim 4,
wherein the first acquisition unit acquires the first information measured at a first time point,
the second acquisition unit acquires the second information estimated by using an estimation model from a first image capturing the bone of the subject at a second time point that is different from the first time point, and
the generator generates the display image in which the first information and the second information are arranged in chronological order of the first time point and the second time point.

6. The information processing system according to claim 5, wherein the estimation model is set based on learning data comprising a second image capturing a bone of a first person and training data comprising at least one selected from the group consisting of a bone density, a bone mass, and a bone quality of the first person.

7. The information processing system according to claim 5 or 6, further comprising a third acquisition unit configured to acquire inference information on at least one selected from the group consisting of a bone density, a bone mass, and a bone quality inferred by using an inference model from an image capturing the bone of the subject at the first time point or the second time point,
wherein,
if the image capturing the bone of the subject is an image capturing the bone of the subject at the first time point, the inference information is information at a third time point that is different from the first time point,
if the image capturing the bone of the subject is an image capturing the bone of the subject at the second time point, the inference information is information at the third time point that is different from the second time point, and
the generator generates the display image showing the inference information associated with the third time point.

8. The information processing system according to claim 7, wherein the inference model is set based on learning data comprising a third image capturing a bone of a second person and training data comprising at least one selected from the group consisting of a bone density, a bone mass, and a bone quality of the second person.

9. The information processing system according to claim 8, wherein
the inference information is information on the bone density of the subject when the subject has received treatment for at least one selected from the group consisting of a bone density, a bone mass, and a bone quality,
the second person is a person who has received treatment for at least one selected from the group consisting of a bone density, a bone mass, and a bone quality, and
the inference model is set based on learning data comprising the third image capturing the bone of the second person who has received the treatment and training data comprising at least one selected from the group consisting of the bone density, the bone mass, and the bone quality of the second person.

10. The information processing system according to any one of claims 7 to 9, wherein,
if the image capturing the bone of the subject is an image capturing the bone of the subject at the first time point, the third time point is a time point before the first time point or a time point after the first time point, and
if the image capturing the bone of the subject is an image capturing the bone of the subject at the second time point, the third time point is a time point before the second time point or a time point after the second time point.

11. The information processing system according to any one of claims 7 to 10, further comprising a fourth acquisition unit configured to acquire third information on the bone of the subject calculated from at least one selected from the group consisting of the first information, the second information, and the inference information,
wherein the generator generates the display image showing the third information.

12. The information processing system according to any one of claims 5 to 11, wherein the generator generates the display image showing information indicating the name of a bone captured in the first image used for estimating the second information.

13. The information processing system according to any one of claims 3 to 12, wherein the generator generates the display image showing the second information in a display mode that is different from a display mode for the first information.

14. The information processing system according to any one of claims 1 to 13, wherein the generator generates the display image showing information indicating a period of treatment for at least one selected from the group consisting of the bone density, the bone mass, and the bone quality of the subject.

15. The information processing system according to any one of claims 1 to 14, wherein the generator generates the display image showing transition information indicating a transition of at least one selected from the group consisting of a bone density, a bone mass, and a bone quality of a reference person who is different from the subject.

16. The information processing system according to any one of claims 1 to 15, wherein the generator generates the display image showing information indicating a determination criterion for at least one selected from the group consisting of a bone density, a bone mass, and a bone quality.

17. The information processing system according to any one of claims 3 to 13, wherein the generator generates the display image comprising display indicating the first method and the second method.

18. The information processing system according to any one of claims 3 to 13, further comprising a measurement apparatus configured to perform measurement for a bone,
wherein the second information comprises information measured using the measurement apparatus.

19. The information processing system according to any one of claims 5 to 12, further comprising an imaging apparatus configured to capture an image of a bone,
wherein the first image is an image captured using the imaging apparatus.

20. An information processing method comprising:
acquiring first information indicating information on a bone of a subject obtained based on a first method;
acquiring second information indicating information on the bone of the subject obtained based on a second method that is different from the first method for the subject; and
generating a display image showing the first information and the second information.

21. An image generation apparatus comprising:
a first acquisition unit configured to acquire first information indicating information on a bone of a subject obtained based on a first method;
a second acquisition unit configured to acquire second information indicating information on the bone of the subject obtained based on a second method that is different from the first method for the subject; and
a generator configured to generate a display image showing the first information and the second information.

22. An image generation program configured to cause a computer to function as the image generation apparatus described in claim 21, the image generation program causing the computer to function as the first acquisition unit, the second acquisition unit, and the generator.
